(19) Europäisches Patentamt
European Patent Office
Office européen des brevets

(11) **EP 4 401 082 A1**

(12) **EUROPEAN PATENT APPLICATION**
published in accordance with Art. 153(4) EPC

(43) Date of publication:
**17.07.2024 Bulletin 2024/29**

(21) Application number: **22864609.7**

(22) Date of filing: **31.08.2022**

(51) International Patent Classification (IPC):
**G16C 20/30** (2019.01)     **G16C 20/40** (2019.01)
**G06F 16/907** (2019.01)

(52) Cooperative Patent Classification (CPC):
**G06F 16/907; G16C 20/30; G16C 20/40**

(86) International application number:
**PCT/JP2022/032725**

(87) International publication number:
**WO 2023/033027 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.09.2021 JP 2021144755**

(71) Applicant: **Resonac Corporation
Tokyo 105-7325 (JP)**

(72) Inventors:
• **MINAMI, Takuya
Tokyo 105-8518 (JP)**
• **HASHIZUME, Naoki
Tokyo 105-8518 (JP)**

(74) Representative: **Strehl Schübel-Hopf & Partner
Maximilianstrasse 54
80538 München (DE)**

(54) **COMPOUND SAFETY PREDICTION DEVICE, COMPOUND SAFETY PREDICTION PROGRAM, AND COMPOUND SAFETY PREDICTION METHOD**

(57)     A compound safety prediction device (1A) according to the present invention comprises: an input unit (10) for inputting the structural formula of at least one molecule; a safety prediction unit (20) for predicting a safety evaluation of the molecule and computing a confidence score level of the prediction; a similar molecule data searching unit (30) for acquiring the safety evaluation data of a similar molecule similar to the molecule; and an output unit (80) for outputting the result of prediction of the safety evaluation of the molecule, the confidence score level of the prediction, and the safety evaluation data of the similar molecule.

FIG.1

**Description**

TECHNICAL FIELD

The present invention relates to compound safety prediction devices, compound safety prediction programs, and compound safety prediction methods.

BACKGROUND ART

There are several tens of millions of kinds of compounds used in chemicals, pharmaceuticals, or the like, and the compounds have various structures. Because a compound may have a harmful effect on the ecosystem and environment, it is extremely important to predict various safety properties, such as a degradability, toxicity, or the like, of the compound. Hence, in various fields, such as the chemical industry, the pharmaceutical industry, or the like, there are studies to develop of a compound safety prediction device for predicting various safeties of the compound.

If a safety prediction rate of the compound is low, the compound has a possibility of harming humans and the environment, and thus, in order to put the safety prediction device into practical use, it is essential to realize an extremely high reliability for the prediction of the compound safety.

As a compound safety prediction device for predicting the safety of the compound, a safety evaluation system which includes a means for learning and analyzing descriptors effective for specific evaluation of a cosmetic material from descriptors computed using information related to cosmetic materials, and a means for searching for an evaluation model effective for the specific evaluation using the analyzed descriptors, and acquiring a prediction value of irritancy, sensitization, or repeated-dose toxicity of the cosmetic material, has been proposed, for example (refer to Patent Document 1, for example).

In addition, as another compound safety prediction device, a method compound safety evaluation method which computes a similarity between a molecule of a general chemical substance with unknown teratogenicity and all pharmaceutical molecules with known teratogenicity prestored in a database, and computes a similarity of a chemical structure that is provided by scores of pharmaceutical safety evaluation related to the molecule of the general chemical substance in a descending order of the scores of the similarity, has been proposed, for example (refer to Patent Document 2, for example).

PRIOR ART DOCUMENTS

PATENT DOCUMENTS

Patent Document 1: Japanese Patent No. 5512077
Patent Document 2: Japanese Laid-Open Patent Publication No. 2007-153767

DISCLOSURE OF THE INVENTION

PROBLEM TO BE SOLVED BY THE INVENTION

However, the technique of Patent Document 1 is limited to prediction of the irritancy, sensitization, or repeated-dose toxicity of the cosmetic material, and there is a problem in that the safety of the compound cannot be predicted with a high accuracy depending on the kind of compound, such as a new compound different from conventional compounds, or the like.

In addition, in the technique of Patent Document 2, there is a problem in that the compound safety evaluation is troublesome to perform and inconvenient for a user, because the similarity must be computed with respect to all of the pharmaceutical molecules registered in the database and the reference must be made to the safety data of the similar molecules.

It is one object of one aspect of the present invention is to provide a compound safety prediction device capable of evaluating the safety of the compound with a high accuracy while improving convenience for the user.

MEANS OF SOLVING THE PROBLEM

The present invention includes the following configurations.

[1] A compound safety prediction device including:

an input unit configured to input a structural formula of one or more molecules;
a safety prediction unit configured to predict a safety evaluation of the molecule and compute a confidence score of prediction;
a similar molecule data searching unit configured to acquire safety evaluation data of a similar molecule similar to the molecule; and
an output unit configured to output a prediction result of the safety evaluation of the molecule, the confidence score of the prediction, and the safety evaluation data of the similar molecule.

[2] The compound safety prediction device according to [1] above, wherein the output unit outputs

a message related to the prediction result of the safety evaluation of the molecule and the confidence score of the prediction, in a case where the confidence score of the prediction is high, and
a message related to the prediction result of the safety evaluation of the molecule, the confidence score of the prediction, and the safety evaluation data of the similar molecule, in a case where the confidence score of the prediction is low.

[3] The compound safety prediction device according to [1] above, further including:
a verification unit configured to verify a validity of the prediction result of the safety evaluation of the molecule from the safety evaluation data of the similar molecule, by determining a coincidence level between the prediction result of the safety evaluation of the molecule and the safety evaluation data of the similar molecule.
[4] The compound safety prediction device according to [3] above, wherein the output unit outputs

a message related to the prediction result of the safety evaluation of the molecule and the confidence score of the prediction in a case where the confidence score of the prediction is high, and
a message related to the prediction result of the safety evaluation of the molecule, the confidence score of the prediction, and the safety evaluation data of the similar molecule, in a case where the confidence score of the prediction is low.

[5] The compound safety prediction device according to [4] above, wherein the output unit, when the confidence score of the prediction is low, outputs

a message indicating that the prediction result of the safety evaluation of the molecule coincides with the safety evaluation data of the similar molecule in a case where the coincidence level is high, and
a message indicating that the prediction result of the safety evaluation of the molecule does not coincide with the safety evaluation data of the similar molecule in a case where the coincidence level is low.

[6] The compound safety prediction device according to any one of [1] to [5] above, wherein the safety prediction unit includes:

a feature value computation unit configured to compute a feature value of the molecule based on the structural formula of the molecule; and
a predictor configured to predict the safety evaluation of the molecule and compute the confidence score of the prediction based on the feature value.

[7] The compound safety prediction device according to [6] above, wherein the feature value computation unit computes the feature value of the molecule using one or more of a fingerprint based on the structural formula of the molecule, a physical property value computed by quantum chemical computation, based on the structural formula of the molecule, a physical property value obtained by a quantitative structure activity relationship between the structural formula and the physical property value of the molecule, and a prediction value obtained by a trained model that has learned the relationship between the structural formula and the physical property value of the molecule.
[8] The compound safety prediction device according to any one [1] to [7] above, wherein the similar molecule data searching unit includes:

a similarity evaluation unit configured to compute a similarity between the structural formula of the molecule input by the input unit and a structural formula of a plurality of evaluated molecules in a safety evaluation database that stores safety evaluation results of the evaluated molecules evaluated in past; and

a data searching unit configured to acquire the safety evaluation result of an evaluated molecule having a high similarity as the safety evaluation data of the similar molecule.

[9] A compound safety prediction program which causes a computer to execute the steps of:

inputting a structural formula of one or more molecules;
predicting a safety evaluation of the molecule and computing a confidence score of prediction;
searching to acquire safety evaluation data of a similar molecule similar to the molecule; and
outputting a prediction result of the safety evaluation of the molecule, the confidence score of the prediction, and the safety evaluation data of the similar molecule.

[10] A compound safety prediction method including the steps of:

inputting a structural formula of one or more molecules;
predicting a safety evaluation of the molecule and computing a confidence score of prediction;
searching to acquire safety evaluation data of a similar molecule similar to the molecule; and
outputting a prediction result of the safety evaluation of the molecule, the confidence score of the prediction, and the safety evaluation data of the similar molecule.

EFFECTS OF THE INVENTION

According to one aspect of the compound safety prediction device, the compound safety prediction program, and the compound safety prediction method according to the present invention, the safety of a compound can be appropriately evaluated by quantifying the confidence score of the prediction of the safety of a molecule, and in a case where the confidence score is high, the safety of the compound can easily be evaluated quickly with a high accuracy by adopting the prediction result as it is. Hence, according to one aspect of the compound safety prediction device, the compound safety prediction program, and the compound safety prediction method according to the present invention, it is possible to evaluate the safety of the compound with a high accuracy, while improving the convenience for the user.

BRIEF DESCRIPTION OF DRAWINGS

[FIG. 1] FIG. 1 is a block diagram illustrating a general configuration of a compound safety prediction device according to a first embodiment of the present invention.
[FIG. 2] FIG. 2 is a diagram illustrating an example of a table in which structural formulas (SMILES) are described.
[FIG. 3] FIG. 3 is an explanatory diagram illustrating an example of a case where a confidence score of prediction is considered to be high when the confidence score of prediction is greater than or equal to 50%.
[FIG. 4] FIG. 4 is a diagram illustrating an example of a table in which prediction results of safety evaluation of molecules are described.
[FIG. 5] FIG. 5 is a diagram illustrating an example of evaluation data of similar molecules.
[FIG. 6] FIG. 6 is a diagram illustrating another example of the evaluation data of the similar molecule.
[FIG. 7] FIG. 7 is a diagram illustrating an example of an integrated file.
[FIG. 8] FIG. 8 is a diagram illustrating an example of a training data table.
[FIG. 9] FIG. 9 is a diagram illustrating an example of feature values of compounds.
[FIG. 10] FIG. 10 is a schematic diagram illustrating a configuration of a model training unit.
[FIG. 11] FIG. 11 is a flow chart for explaining a model training method.
[FIG. 12] FIG. 12 is a flow chart for explaining a compound safety prediction method according to the first embodiment of the present invention.
[FIG. 13] FIG. 13 is a flow chart for explaining a checking process (step S22) in FIG. 12.
[FIG. 14] FIG. 14 is a flow chart for explaining a computation process (step S23) to predict safety evaluation of a molecule and compute a confidence score of the prediction in FIG. 12.
[FIG. 15] FIG. 15 is a flow chart for explaining a searching process (step S24) to search for safety evaluation data of the similar molecule in FIG. 12.
[FIG. 16] FIG. 16 is a flow chart for explaining an integration process (step S25) in FIG. 12.
[FIG. 17] FIG. 17 is a block diagram illustrating a general configuration of the compound safety prediction device according to a second embodiment of the present invention.
[FIG. 18] FIG. 18 is a flow chart for explaining the compound safety prediction method according to the second embodiment of the present invention.
[FIG. 19] FIG. 19 is a block diagram illustrating a hardware configuration of the compound safety prediction device.

MODE OF CARRYING OUT THE INVENTION

Hereinafter, embodiments of the present invention will be described in detail. In order to facilitate understanding of the description, the same constituent elements are designated by the same reference numerals in each of the drawings, and a redundant description thereof will be omitted. In addition, in the present specification, "to" indicating a numerical range, includes numerical values appearing before and after the "to", as a lower limit value and an upper limit value of the range, unless indicated otherwise.

[First Embodiment]

<Compound Safety Prediction Device >

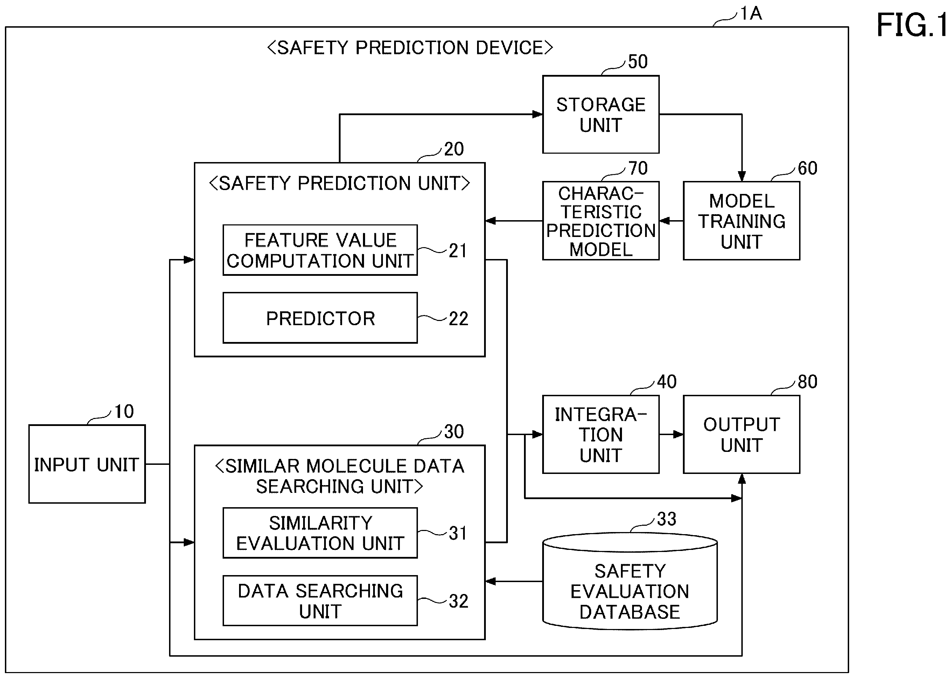

A compound safety prediction device according to a first embodiment of the present invention will be described. FIG. 1 is a block diagram illustrating a general configuration of the compound safety prediction device according to the present embodiment. As illustrated in FIG. 1, a compound safety prediction device (hereinafter simply referred to as a "safety prediction device") 1A includes an input unit 10, a safety prediction unit 20, a similar molecule data searching unit 30, an integration unit 40, a storage unit 50, a model training unit 60, a characteristic prediction model 70, and an output unit 80.

The safety prediction device 1A outputs a prediction result of a molecule safety evaluation and a confidence score of the prediction obtained by the safety prediction unit 20, and safety evaluation data obtained by the similar molecule data searching unit 30. Accordingly, a device user (user) can consider whether or not to adopt the prediction result as it is when the confidence score is high, and whether or not to adopt either the prediction result or the safety evaluation data when the confidence score is low. Hence, the safety prediction device 1A quantifies the confidence score and outputs the quantified confidence score, so that the user can judge the safety of the compound, based on at least one of the prediction result of the molecule safety evaluation obtained by the safety prediction unit 20 and the safety evaluation data obtained by the similar molecule data searching unit 30. Thus, the safety prediction device 1A can improve the convenience for the user, and improve the accuracy of the compound safety evaluation.

The term output includes displaying on a screen, generating sound, or the like, as will be described later.

The confidence score that is high and the confidence score that are low are the same as a high confidence score and a low confidence score which will be described later, respectively, and a threshold value for determining whether the confidence score is high or low can be set appropriately according to a type of molecule to be evaluated for safety. For example, when the threshold value is set to 50%, the confidence score is determined to be high if the confidence score is greater than or equal to the threshold value.

The safety is an index indicating a magnitude of a load of a compound on humans and the environment, and examples of the safety include biodegradability, bioaccumulation, mutagenicity, acute toxicity, acute immobilization, growth inhibition, repeated-dose toxicity, or the like.

The input unit 10 inputs a structural formula of one or more molecules for which the safety is to be evaluated.

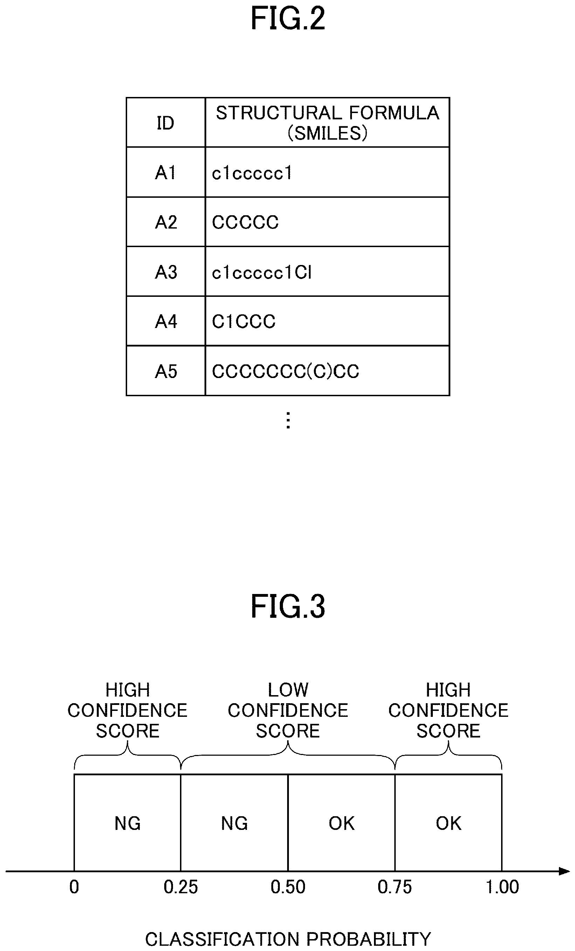

SMILES or the like can be used as the structural formula. The SMILES is a character string representing a molecular structure of a compound. An example of a table describing the structural formulas (SMILES) is illustrated in FIG. 2. As illustrated in FIG. 2, the SMILES represents each of the compounds by assigning ID numbers A1, ... to each of the compounds. The table including the structural formula of each of the molecules may be obtained from data in a format, such as CSV, spreadsheet software such as Excel, or the like. The input unit 10 may input a table in which the SMILES of each of the molecules are described as illustrated in FIG. 2.

The input unit 10 may check the input structural formula of the molecule for a description error. When the user inputs the structural formula, there is a possibility of making an erroneous input. The input unit 10 can determine that the structural formula of the molecule that is input includes a description error by checking the erroneous input of the structural formula.

The input unit 10 may determine whether or not the input structural formula of the molecule includes a description error, by checking whether or not the input structural formula of the molecule is converted into a molecule Mol object, using RDKit or the like included in a library, such as Anaconda (registered trademark), which is software distributed from Anaconda, Inc. of the United States, or the like, for example. In a case where the structural formula is the SMILES, MolFromSmiles included in the RDKit is used to read the character string of the SMILES and read the structural formula of the molecule. In a case where the SMILES is converted into the Mol object and the molecule Mol object is normally created, it can be determined that there is no description error in the input structural formula of the molecule. On the other hand, in a case where the SMILES is not converted into the Mol object and the molecule Mol object is not created, it can be determined that the input structural formula of the molecule is erroneous.

The input unit 10 may separately create a table including the structural formulas having no description error and a table including the structural formulas having a description error, and output the tables by the output unit 80 which will be described later. Thus, even in a case where the user fails to input the structural formula correctly and the structural formula includes an error, the safety prediction device 1A can predict a safety evaluation without abnormally ending the prediction.

As illustrated in FIG. 1, the safety prediction unit 20 predicts the safety evaluation of the molecule, and computes the confidence score of the prediction. The safety prediction unit 20 includes a feature value computation unit 21, and a predictor 22.

The feature value computation unit 21 computes a feature value based on the structural formula of the molecule.

The feature value can be obtained based on the structural formula of the molecule having no description error. Fingerprints based on the structural formulas of the molecules, such as fingerprints corresponding to EXTENDED Connectivity Fingerprints (ECFP) computed using Morgan fingerprints (Circular fingerprints) implemented in the RDKit, other fingerprints such as AtomPair or the like, or the like can be used as the feature value. The feature value may be a physical property, such as an octanol/water partition coefficient (logP) or the like, representing a liposolubility of the molecule. The fingerprint may express the presence and the absence of a partial structure by 1 and 0, respectively, express a number of partial structures, or express a ratio of partial structures obtained by dividing the number of partial structures by a number of constituent atoms.

The feature value may be computed using one or more of a physical property value computed by quantum chemical computation based on the structural formula of the molecule, a physical property value obtained by a quantitative structure activity relationship between the structural formula and the physical property value of the molecule, and a prediction value obtained by a trained model that has learned the relationship between the structural formula and the physical property value of the molecule. Examples of the physical property value computed by the quantum chemical computation include HOMO, LUMO, electric charge, refractive index, frequency, or the like. The structure activity relationship refers to a correlation between the chemical structural feature (or a physicochemical constant) of a substance, and the biological activity (for example, the degradability, accumulation, various toxicity endpoints, or the like).

In addition, the feature value may be a physical property measurable by an experiment on a melting point, viscosity, and a specific surface area, or the like.

The predictor 22 predicts the safety evaluation of the molecule based on the feature value computed by the feature value computation unit 21, and computes the confidence score of the prediction.

For example, a biochemical oxygen demand (BOD) or the like can be used as an index for evaluating the safety of the molecule. In a case where the BOD is greater than or equal to a predetermined value (for example, 60%), the safety of the molecule can be evaluated as being good.

The confidence score of the prediction can be computed using the characteristic prediction model 70. The predictor 22 inputs the feature value computed by the feature value computation unit 21, as an explanatory variable, to the characteristic prediction model 70, and outputs a classification probability P(OK) that indicates a classification result "OK", where OK indicates good. The predictor 22 computes a confidence score (unit: %) of the prediction, with respect to a classification probability P(OK) for which the classification result is "OK", using the following formula (1).

$$\mathtt{Certainty\ Factor\ of\ Prediction\ (\%)}$$
$$\equiv 100 \times 2 \times |0.5 - P(OK)| \qquad\qquad --- (1)$$

(In the formula (1), P(OK) indicates a classification probability for which the classification result is "OK".)

The confidence score of the prediction takes a value from 0% to 100%, and a percentage of correct answer of the prediction result becomes higher as the confidence score of the prediction becomes closer to 100%. For this reason, the user can easily judge, from the confidence score of the prediction, whether or not the prediction result is reliable.

The confidence score of the prediction corresponds to the classification probability as in the formula (1) described above, and the confidence score of the prediction varies according to a magnitude of the classification probability. FIG. 3 illustrates an example of a case where the confidence score of the prediction is considered to be high when the confidence score of the prediction is greater than or equal to 50%. In a case where the classification probability is greater than or equal to 0 and less than or equal to 0.25, the confidence score of the prediction becomes greater than or equal to 50% and less than or equal to 100%, and the prediction is regarded as "NG high confidence score" where NG indicates not good. In a case where the classification probability is greater than 0.25 and less than 0.50, the confidence score of the prediction becomes greater than 0% and less than 50%, and the prediction is regarded as "NG low confidence score". In a case where the classification probability is greater than or equal to 0.50 and less than or equal to 0.75, the confidence score of the prediction becomes greater than or equal to 0% and less than 50%, and the prediction is regarded as "OK low confidence score". In a case where the classification probability is greater than or equal to 0.75 and less than or equal to 1.00, the confidence score of the prediction becomes greater than or equal to 50% and less than or equal to 100%, and the prediction is regarded as "OK high confidence score". By quantifying the confidence score of the prediction in this manner, the user can easily judge the reliability of the prediction result.

The threshold values for determining the high confidence score and the low confidence score can be set appropriately according to the type of molecule to be evaluated for safety, and are preferably 50%, respectively, for example.

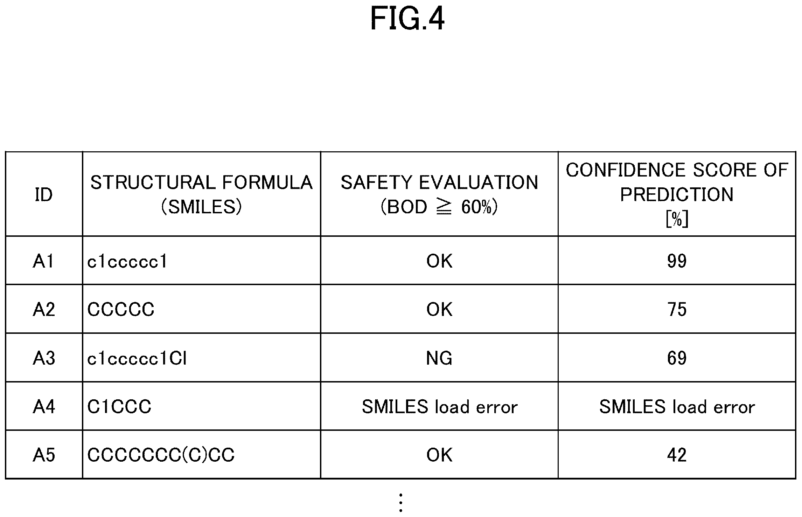

The predictor 22 can create a table of the prediction result of the safety evaluation of the molecule, including the structural formula of each molecule, the prediction result, and the confidence score of the prediction. An example of the table describing the prediction result of the safety evaluation of the molecule is illustrated in FIG. 4. In FIG. 4, the SMILES are used for the structural formulas of the molecules, and the ID numbers A1, ... of each of the compounds and the SMILES of each of the compounds are used. The BOD is used as an index for evaluating the safety of the molecule. In a case where the BOD is greater than or equal to 60%, the safety evaluation of the molecule is good (OK), and in a case where the BOD is less than 60%, the safety of the molecule is not good (NG) .

As illustrated in FIG. 4, the table of the prediction result of the safety evaluation of the molecule can include the prediction result of the safety evaluation of the molecule, and the confidence score of the prediction, for each of the molecules and each of the SMILES thereof, obtained by the safety prediction unit 20. In a case where an erroneous input is included in the SMILES, "SMILES load error" is displayed, and information indicating the erroneous input and the state where the SMILES of the compound cannot be recognized is output. When the confidence score of the prediction of the safety evaluation is high as in the case of the IDs A1 to A3, this prediction may be regarded as being reliable. On the other hand, when the confidence score of the prediction is low as in the case of the ID A5, this prediction may be regarded as being unreliable. When the confidence score of the prediction is low, the user can evaluate the safety of the molecule for the safety evaluation in more detail, by referring to a result of a search for a similar molecule performed by the similar molecule data searching unit 30 which will be described later.

The feature value computation unit 21 may create a table of the prediction result of the safety evaluation of the molecule, including the prediction result of the safety evaluation of the molecule and the confidence score of the prediction, as illustrated in FIG. 4, and output the table from the output unit 80 which will be described later. Hence, the user can ascertain the prediction result related to the safety evaluation of the molecule in a simple manner.

As illustrated in FIG. 1, the similar molecule data searching unit 30 acquires safety evaluation data of the similar molecule that is similar to the molecule to be evaluated. The similar molecule data searching unit 30 includes a similarity evaluation unit 31, and a data searching unit 32.

The similarity evaluation unit 31 computes and evaluates a similarity between the structural formula of the molecule input from the input unit 10, and the structural formulas of the plurality of evaluated molecules stored in a safety evaluation database 33. The similarity evaluation unit 31 may use the SMILES for the structural formula of the molecule.

The safety evaluation database 33 stores safety evaluation data of the evaluated molecules evaluated in the past.

The similarity can be obtained by computing a Tanimoto coefficient, using Bulk Tanimoto Similarity implemented in the RDKit. The similarity may be a Dice coefficient, a cosine (cos) similarity, or the like.

The similarity evaluation unit 31 can vary the number of safety evaluation data of similar molecules to be acquired as appropriate according to the purpose, ease of use, or the like, among the safety evaluation data stored in the safety evaluation database 33, and may acquire a predetermined number of data corresponding to top similarities (for example, top 20 data), as the safety evaluation data of the similar molecule (similar molecule data) .

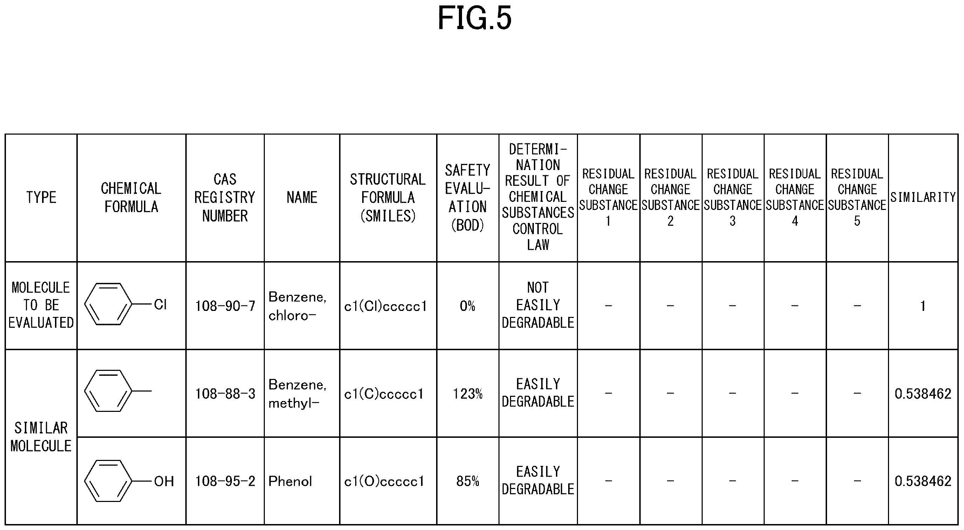

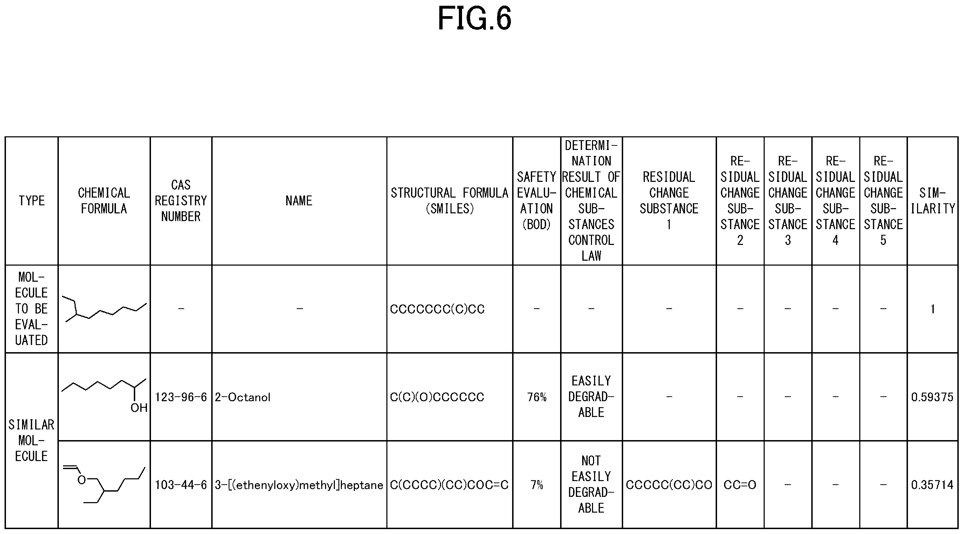

As illustrated in FIG. 5 and FIG. 6, examples of the safety evaluation data of the similar molecule include, as information on these molecules, a chemical formula of the molecule, a CAS registry number, name of the molecule, a structural formula (SMILES), safety evaluation (BODs), a determination result of the Chemical Substances Control Law, residual change substances 1 to 5, and similarities, for example.

The determination by the Chemical Substances Control Law refers to a determination by the "Act on the Regulation of Manufacture and Evaluation of Chemical Substances".

The residual change substance refers to a change substance remaining after a test of a biodegradability test in conformance with the Chemical Substances Control Law or the like.

As illustrated in FIG. 5, in the table including the safety evaluation data of the similar molecules, a first row indicates the information of the molecule having the ID A3 in FIG. 4 as the molecule to be evaluated, and second and subsequent rows indicate the information of the similar molecules recorded in past data. By referring to the molecule to be evaluated indicated in the first row, it is possible to confirm whether or not the molecule to be evaluated is not easily degradable. Then, by comparing the molecule to be evaluated indicated in the first row with the similar molecules indicated in the second and subsequent rows, the user can confirm whether the molecule is easily degradable or not easily degradable.

As illustrated in FIG. 6, in another example of the table including the safety evaluation data of the similar molecule, the first row indicates the structural formula of the molecule having the ID A5 in FIG. 4 as the molecule to be evaluated, and the second and subsequent rows indicate the information of the similar molecules recorded in the past data. By comparing the information of the molecule to be evaluated indicated in the first row with the similar molecules indicated in the second and subsequent rows, the user can confirm whether the similar molecule is easily degradable or not easily degradable, even if the degradability of the molecule to be evaluated indicated in the first row cannot be confirmed, and thus, it is easy to determine whether the molecule to be evaluated is easily degradable or not easily degradable.

The similarity evaluation unit 31 displays information on the molecule to be evaluated, and information related to the similar molecules, together in the table including the safety evaluation data of the similar molecule, so that the molecule to be evaluated and the similar molecules can be compared visually, and thus, the user can easily determine the safety evaluation data of the similar molecule to be referred to among the similar molecules.

The similarity evaluation unit 31 may create a table including the safety evaluation data of the similar molecule, such as that illustrated in FIG. 5 and FIG. 6, and output the table from the output unit 80 which will be described later. In this case, the user can ascertain the information related to the similar molecules.

The data searching unit 32 acquires the safety evaluation data of the similar molecule having a high similarity.

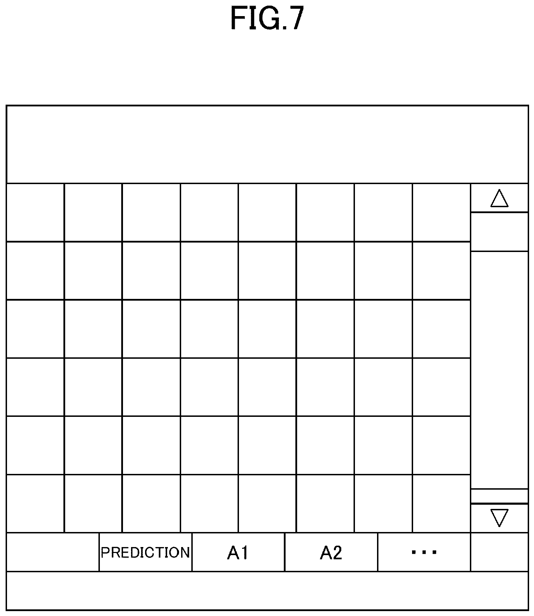

As illustrated in FIG. 1, the integration unit 40 integrates a prediction result file including a prediction result of the safety evaluation of the molecule to be evaluated and a confidence score of the prediction obtained by the safety prediction unit 20, and an evaluation data file including the safety evaluation data obtained by the similar molecule data searching unit 30. As a result, the integration unit 40 creates an integrated file in which the prediction result file (refer to FIG. 4) obtained by the safety prediction unit 20 and the evaluation data file (refer to FIG. 5 and FIG. 6) obtained by the similar molecule data searching unit 30 are integrated, as illustrated in FIG. 7. In FIG. 7, contents of the prediction result file are described in a prediction sheet, and the evaluation data files of the similar molecules having the respective IDs are described in an A1 sheet, an A2 sheet, ..., for example.

The integration unit 40 may output the integrated file from the output unit 80 which will be described later. In this case, the user can easily ascertain together the information related to the molecule to be evaluated and the information related to the safety evaluation of the similar molecules, which are included in the integrated file.

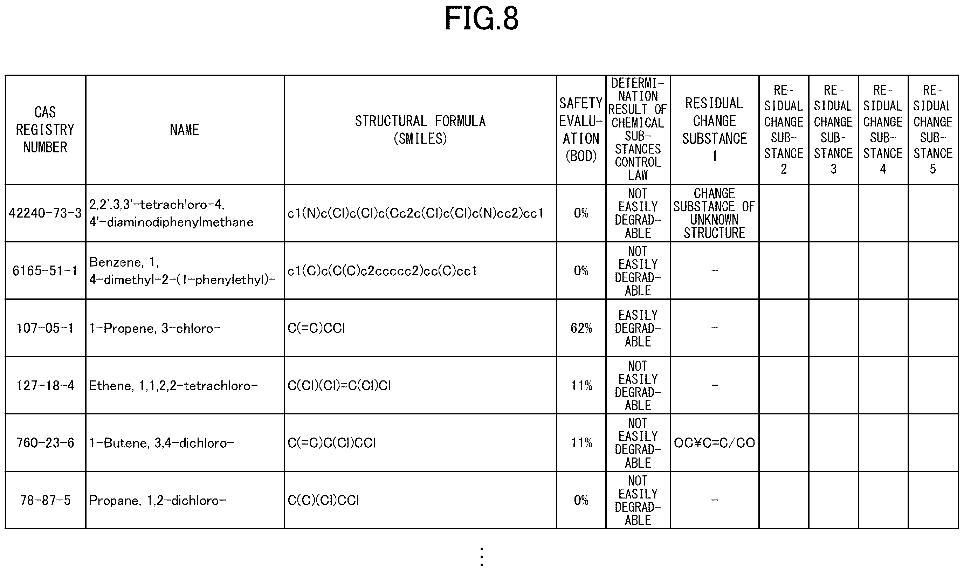

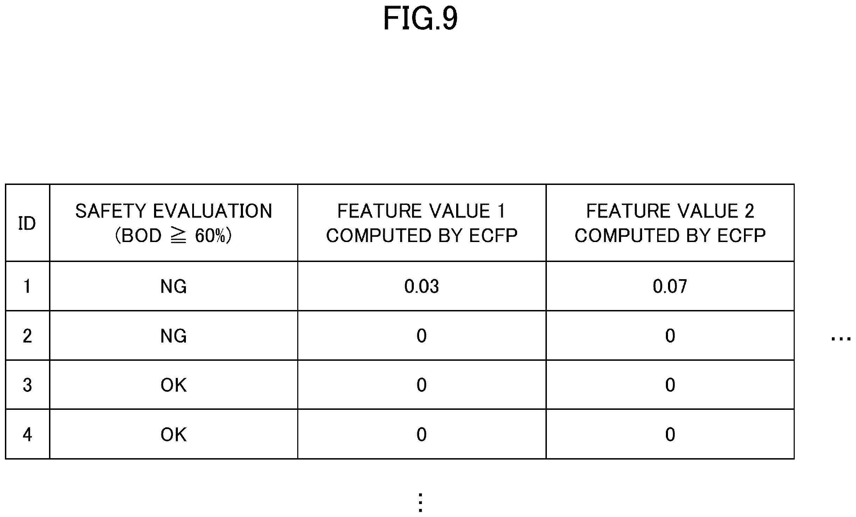

The storage unit 50 stores, as training data, associated data in which the structural formula of the molecule of the compound, the safety evaluation, the feature value of the compound, the characteristics of the compound, or the like are associated with one another. An example of the training data table is illustrated in FIG. 8. As illustrated in FIG. 8, the training data includes a correspondence relationship among the CAS registry number, the SMILES, the BOD that is the safety evaluation result as an objective variable of the compound, the determination result of the Chemical Substances Control Law as the characteristics of the compound, the type of the residual change substance, or the like of the molecule of the compound. In FIG. 8, "-" indicates "not applicable". The feature value of the compound is computed from the SMILES of the corresponding compound by a technique, such as the ECFP or the like. For example, as illustrated in FIG. 9, the feature value of the compound is represented in a matrix format of numerical values, as feature values 1, 2, or the like computed by the ECFP.

The storage unit 50 may update the associated data by inputting the structural formula (for example, the SMILES or the like) of the molecule of the compound, the feature value of the compound, the characteristics of the compound, or the like to the associated data.

The model training unit 60 trains a model by utilizing the associated data stored in the storage unit 50 as the training data.

More particularly, the model training unit 60 uses the structural formula (for example, the SMILES or the like) of the molecule of the compound and the feature value of the compound, stored in the storage unit 50, as an explanatory variable, and uses the characteristics of the compound to be predicted as the objective variable. Thus, the model training unit 60 trains the model for specifying the correspondence relationship between the feature value of the compound and the characteristics of the compound, and generates a trained model (characteristic prediction model 70). The model training unit 60 trains the model, so that the correspondence relationship approaches the correspondence relationship of the training data by machine learning.

The machine learning applied to the model is preferably an algorithm of supervised learning. Examples of the supervised learning include linear regression, logistic regression, random forest, boosting, support vector machine (SVM), neural network, or the like, for example. The neural network may use deep learning (deep learning) in which a multi-layer neural network is formed of more than three layers. Examples of the type of the neural network include a convolutional neural network (CNN), a recurrent neural network (RNN), a general regression neural network, or the like, for example. In addition, the model may be expressed by a mathematical expression, such as a function or the like.

More particularly, a machine trained model constructed using Anaconda (registered trademark) or the like, which is software distributed from Anaconda, Inc. of the United States.

Anaconda (registered trademark) includes library groups used by machine learning, such as scikit-learn or the like, and the model training unit 60 may perform machine learning using one or more of such libraries.

Further, the model training unit 60 may perform relearning on the trained model by using, from the safety evaluation data newly stored in the storage unit 50, the structural formula (for example, the SMILES or the like) of the molecule of the compound and the feature value of the compound as explanatory variables, and the characteristics of the compound as objective variables.

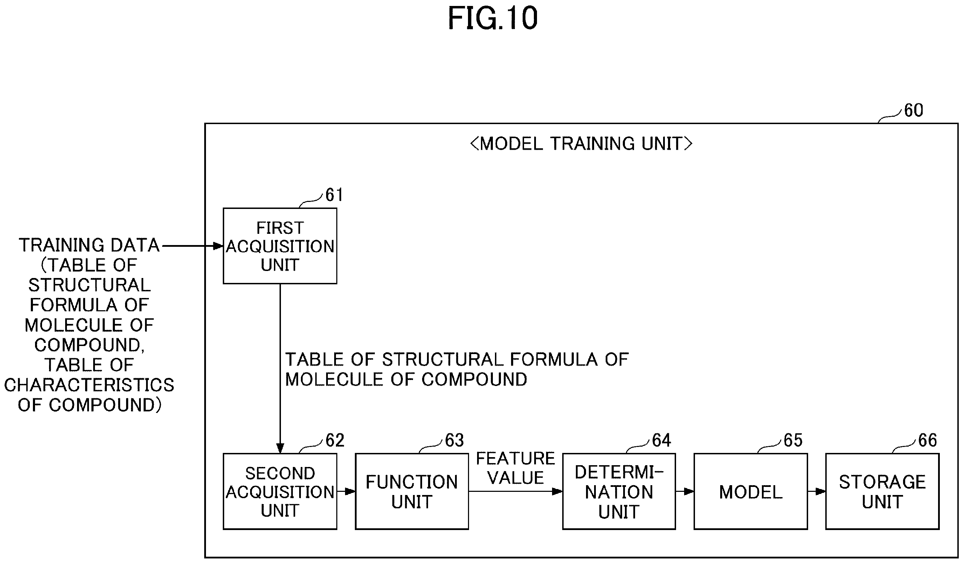

FIG. 10 is a schematic diagram illustrating a configuration of the model training unit 60. As illustrated in FIG. 10, the model training unit 60 includes a first acquisition unit 61, a second acquisition unit 62, a function unit 63, a determination unit 64, a model 65, and a storage unit 66.

The first acquisition unit 61 acquires the training data including a table that includes the structural formula (for example, the SMILES) of the molecule of the compound and a listing of the structural formulas, and a table including a listing of the characteristics of the compound.

The training data can be stored in a file having a format, such as CSV, Excel which is spreadsheet software, or the like, for example.

The second acquisition unit 62 acquires the molecular structure of one molecule, from the training data acquired by the first acquisition unit 61.

The molecular structure of one molecule is preferably the SMILES of one molecule.

The function unit 63 computes the feature value, based on the molecular structure of one molecule acquired by the second acquisition unit 62. Because the feature can be computed in a manner similar to the feature value computation unit 21, a detailed description thereof will be omitted.

The determination unit 64 determines whether or not the feature values of all molecules included in the training data are computed.

The model 65 is trained by the model training unit 60, using the structural formula of the molecule of the compound and the feature value of the compound stored in the storage unit 50 as explanatory variables, and the characteristics of the compound stored in the storage unit 50 as objective variables.

The storage unit 66 stores the trained model generated by the model training unit 60 which trains the model 65.

As illustrated in FIG. 1, the characteristic prediction model 70 is the trained model generated by the model training unit 60 which trains the model 65.

The high and low levels of the confidence score of the prediction may be appropriately set according to the predetermined value of the classification probability. In a case where the predetermined value of the classification probability is 0.50, the high confidence score of the prediction refers to a case where the confidence score of the prediction is greater than or equal to 50%, for example, and the low confidence score of the prediction refers to a case where the confidence score of the prediction is less than 50%, for example.

The output unit 80 outputs the prediction result of the safety evaluation of the molecule, the confidence score of the prediction, and the safety evaluation data of the similar molecule obtained by the integration unit 40. That is, the output unit 80 outputs the integrated file.

The output may utilize any method capable of notifying the user, and includes displaying on a screen, generating sound, or the like.

In addition, the output unit 80 may output a table of structural formulas (for example, the SMILES) having no description error, and a table of structural formulas having a description error, which are created by the input unit 10. Moreover, the output unit 80 may output a table of the prediction results of the safety evaluation of the molecules, including the prediction results of the safety evaluation of the molecules and the confidence scores of the prediction, which is created by the safety prediction unit 20, or may output the safety evaluation data of the similar molecule, including information related to the similar molecules, which is created by the similarity evaluation unit 31. Further, the output unit 80 may refer to the integrated file, and output the safety evaluation data of the similar molecule when the confidence score of the prediction of the safety evaluation of the molecule is low.

The output unit 80 may output a message related to the prediction result of the safety evaluation of the molecule and the confidence score of the prediction in a case where the confidence score of the prediction of the safety evaluation of the molecule is high (high confidence score), and output a message related to the prediction result of the safety evaluation of the molecule, the confidence score of the prediction, and the safety evaluation data of the similar molecule in a case where the confidence score of the prediction of the safety evaluation of the molecule is low (low confidence score).

For example, the contents of the message may be "The prediction result of the safety evaluation of the molecule is high, and the confidence score of the prediction is greater than or equal to 50%." or the like in the case where the confidence score of the prediction is high, and may be "The prediction result of the safety evaluation of the molecule is low, and the confidence score of the prediction is less than 50%." or the like in the case where the confidence score of the prediction is low.

<Compound Safety Prediction Program>

A compound safety prediction program according to the present embodiment (hereinafter, simply referred to as a "safety prediction program") can use a program having the following configuration.

That is, the safety prediction program according to the present embodiment may use a program which causes a computer to execute at least the steps of:

> inputting a structural formula of one or more molecules;
> predicting a safety evaluation of the molecule and computing a confidence score of prediction;
> searching to acquire safety evaluation data of a similar molecule similar to the molecule; and
> outputting a prediction result of the safety evaluation of the molecule, the confidence score of the prediction, and the safety evaluation data of the similar molecule.

<Compound Safety Prediction Method >

Next, a compound safety prediction method (hereinafter, simply referred to as a "safety prediction method") applied to the safety prediction device according to the present embodiment will be described. The safety prediction method applied to the safety prediction device according to the present embodiment is a method for predicting the safety evaluation of the compound using the safety prediction device 1A having the configuration illustrated in FIG. 1.

Next, a training method of the characteristic prediction model 70 used in the safety prediction method will be described. As described above, because the model 65 constructed by the model training unit 60 is applied to the characteristic prediction model 70, the training method of the characteristic prediction model 70 will be described as the training method of the model 65.

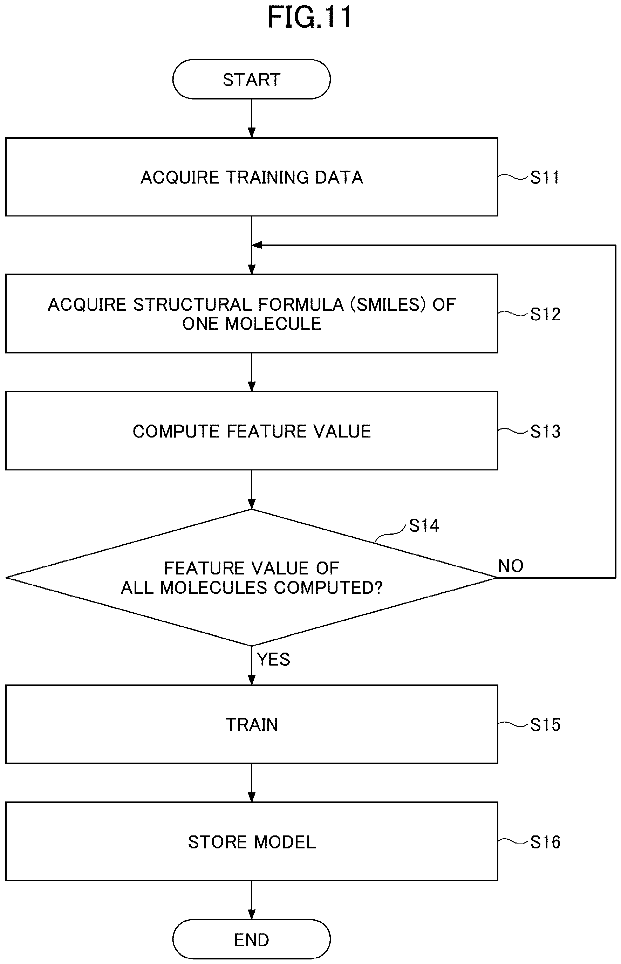

FIG. 11 is a flow chart for explaining the model training method. As illustrated in FIG. 11, the model training method is a method of training the model in the model training unit 60 having the configuration illustrated in FIG. 10, using training data in which the explanatory variables, including the structural formula of the molecule of compound and the feature value of the compound stored in the storage unit 50 of the safety prediction device 1A illustrated in FIG. 1, are associated with the objective variables, including characteristics of the compound stored in the storage unit 50 of the safety prediction device 1A.

In the model training method, the first acquisition unit 61 of the safety prediction device 1A acquires the training data (training data acquisition process: step S11).

The training data includes a table listing the structural formulas (for example, the SMILES) of the molecules of the compounds, a table listing the characteristics of the compounds, or the like.

Next, the second acquisition unit 62 of the safety prediction device 1A acquires the structural formula of one molecule from the training data (acquisition process to acquire the structural formula of one molecule: step S12).

The structural formula of one molecule may be the SMILES of one molecule.

Next, the second acquisition unit 62 of the safety prediction device 1A computes the feature value by utilizing a library group included in Anaconda (registered trademark), such as scikit-learn and RDKit or the like, using the structural formula of one molecule acquired by the function unit 63 (feature value computation process: step S13).

Next, the determination unit 64 of the safety prediction device 1A determines whether or not the feature value of all of the molecules included in the training data are computed (determination process to determine the feature value of all of the molecules: step S14).

If the feature value of all of the molecules are not computed (step S14: No), the process returns to the acquisition process to acquire the structural formula of one molecule (step S12), and the structural formula of the remaining molecule for which the feature value is not computed is acquired.

If the feature value of all of the molecules is computed (step S14: Yes), the model training unit 60 trains the model using training data in which the explanatory variables including the feature value of all of the molecules and the objective variables including the characteristics of all of the molecules are associated with one another, and constructs the model 65 (training process: step S15).

The training unit 15 trains the model so that the output coincides with the objective variable associated with the explanatory variable, according to the input of the explanatory variable included in the training data.

Next, the safety prediction device 1A stores the model constructed by the training unit 15 in the storage unit 66 (storing process: step S16).

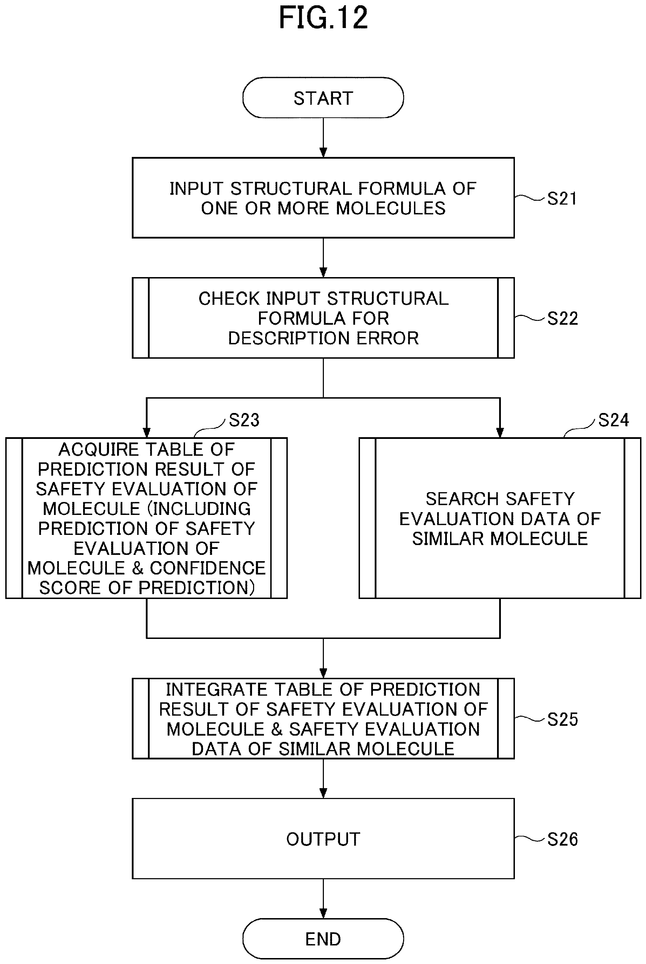

Next, the safety prediction method applied to the safety prediction device according to the present embodiment will be described. FIG. 12 is a flow chart for explaining the safety prediction method according to the present embodiment. As illustrated in FIG. 12, the input unit 10 of the safety prediction device 1A inputs the structural formula of one or more molecules to be evaluated for safety (input process: step S21).

Next, the safety prediction unit 20 of the safety prediction device 1A checks the input structural formula for a description error (checking process: step S22) .

The details of the checking process (step S22) will be described later. The checking process (step S22) may be omitted.

Next, the safety prediction unit 20 of the safety prediction device 1A predicts the safety evaluation of the molecules and computes the confidence score of the prediction, and acquires a table of the prediction result of the safety evaluation of the molecules, including the prediction of the safety evaluation of the molecule and the confidence score of the prediction (computation process to predict the safety evaluation of the molecule and compute the confidence score of the prediction: step S23).

The details of the computation process (step S23) to predict the safety evaluation of the molecule and compute the confidence score of the prediction will be described later.

The similar molecule data searching unit 30 of the safety prediction device 1A searches and acquires the safety evaluation data of the similar molecule similar to the molecule to be evaluated for safety (searching process to search and acquire the safety evaluation data of the similar molecule: step S24).

The details of the searching process (step S24) to search and acquire the safety evaluation data of the similar molecule will be described later.

Next, the integration unit 40 of the safety evaluation prediction device 1A obtains the integrated data by integrating the prediction result of the safety evaluation of the molecule and the confidence score of the prediction obtained in the computation process (step S23) to predict the safety evaluation of the molecule and compute the confidence score of the prediction, and the safety evaluation data of the similar molecule acquired in the searching process (step S24) to search and acquire the safety evaluation data of the similar molecule (integration process: step S25).

The details of the integration process (step S25) will be described later.

Next, the output unit 80 of the safety prediction device 1A outputs the integrated data integrated by the integration unit 40 (output process:

step S26).

The safety prediction device 1A may output, from the output unit 80, the prediction result and the confidence score of the prediction of the integrated data by making a display or the like in the case where the confidence score of the prediction is high, and output the safety evaluation data of the similar molecule in addition to the prediction result and the confidence score of the prediction of the integrated data by making a display or the like in the case where the confidence score of the safety prediction is low.

The computation process (step S23) to predict the safety evaluation of the molecule and compute the confidence score of the prediction may be performed simultaneously with the searching process (step S24) to search and acquire the safety evaluation data of the similar molecule, or may be performed after the searching process (step S24) to search and acquire the safety evaluation data of the similar molecule.

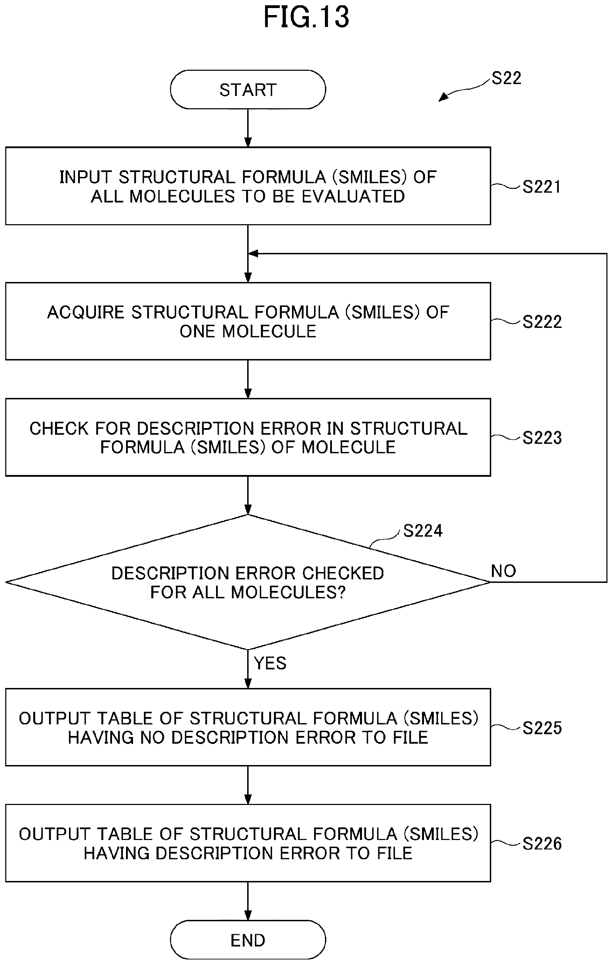

Next, the checking process (step S22) in FIG. 12 will be described. FIG. 13 is a flow chart for explaining the checking process (step S22) in FIG. 12. As illustrated in FIG. 13, the safety prediction unit 20 of the safety prediction device 1A inputs the structural formula of all of the molecules to be evaluated for the safety (input process to input the structural formula of all of the molecules to be evaluated: step S221).

The SMILES illustrated in FIG. 2 may be acquired as the structural formulas of the molecules, for example.

Next, the safety prediction unit 20 of the safety prediction device 1A acquires the structural formula of one molecule from among the input structural formulas of all of the molecules to be evaluated (acquisition process to acquire the structural formula of one molecule: step S222).

Next, the safety prediction unit 20 of the safety prediction device 1A checks for a description error in the structural formula of one molecule (description error checking process: step S223).

Next, the safety prediction unit 20 of the safety prediction device 1A determines whether or not the computation error of the structural formula is checked for all of the molecules (description error determination process: step S224).

If the computation error is not determined for all of the molecules (step S224: No), the structural formula of the molecule not determined of the description error is acquired again (step S222).

If the computation error is determined for all of the molecules (step S224: Yes), the safety prediction unit 20 of the safety prediction device 1A outputs the table of the structural formula having no description error to a file (output process to output the table of structural formulas having no description error: step S225).

Next, the safety prediction unit 20 of the safety prediction device 1A outputs the table of the structural formula having the description error to a file (output process to output the structural formula having the description error: step S226).

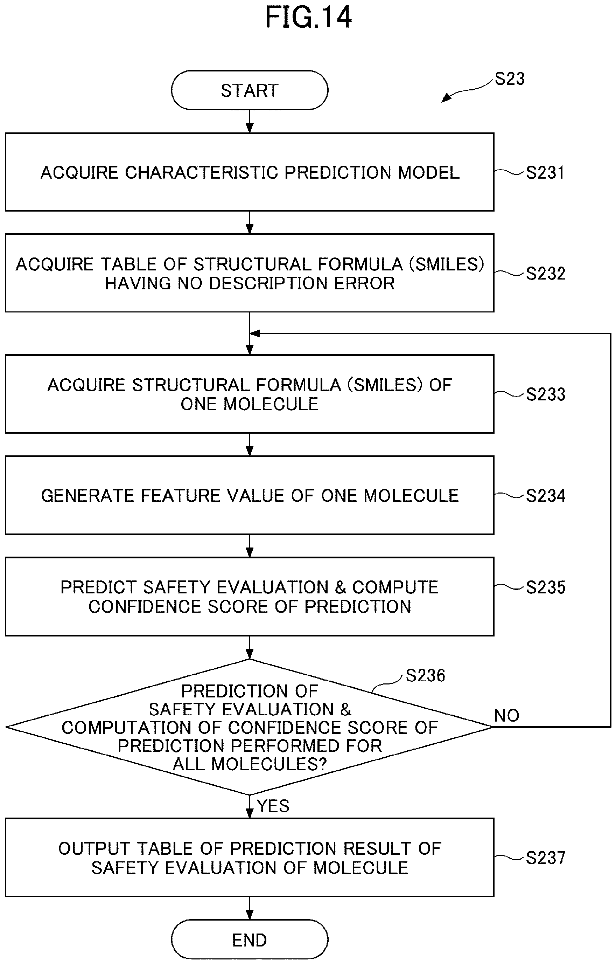

Next, the computation process (step S23) to predict the safety evaluation of the molecule and compute the confidence score of the prediction in FIG. 12 will be described. FIG. 14 is a flow chart for explaining the computation process (step S23) to predict the safety evaluation of the molecule and compute the confidence score of the prediction in FIG. 12. As illustrated in FIG. 14, the safety prediction unit 20 of the safety prediction device 1A acquires the model obtained by the model training unit 60 as the characteristic prediction model 70 (characteristic prediction model acquisition process: step S231).

Next, the safety prediction unit 20 of the safety prediction device 1A acquires the table of the structural formula having no description error (structural formula acquisition process: step S232).

Next, the safety prediction unit 20 of the safety prediction device 1A acquires the structural formula of one molecule among the structural formulas of all of the molecules described in the table of the structural formulas having no description error (acquisition process to acquire the structural formula of one molecule: step S233).

Next, safety prediction unit 20 of the safety prediction device 1A generates a feature value of one molecule (generation process to generate feature value of one molecule: S234).

Next, the safety prediction unit 20 of the safety prediction device 1A predicts the safety evaluation of one molecule and computes the confidence score of the prediction (computation process to predict safety evaluation of molecule and compute confidence score of prediction: S235).

Next, the safety prediction unit 20 of the safety prediction device 1A determines whether or not the prediction of the safety evaluation and the computation of the confidence score of the prediction are performed for all of the molecules (determination process to determine the prediction of the safety evaluation and the computation of the confidence score of the prediction for all of the molecules: step S236).

If the prediction of the safety evaluation and the computation of the confidence score of the prediction are not performed for all of the molecules (step S236: No), the structural formula of the molecule for which the prediction and computation are not performed is acquired again (step S232).

If the prediction of the safety evaluation and the computation of the confidence score of the prediction are performed for all of the molecules (step S236: Yes), the table of the prediction results of the safety evaluation of the molecules, including the prediction of the safety evaluation for all of the molecules and the confidence score of the prediction of all the molecules is output to a file (output process to output the table of prediction results of the safety evaluation of the molecules: step S237).

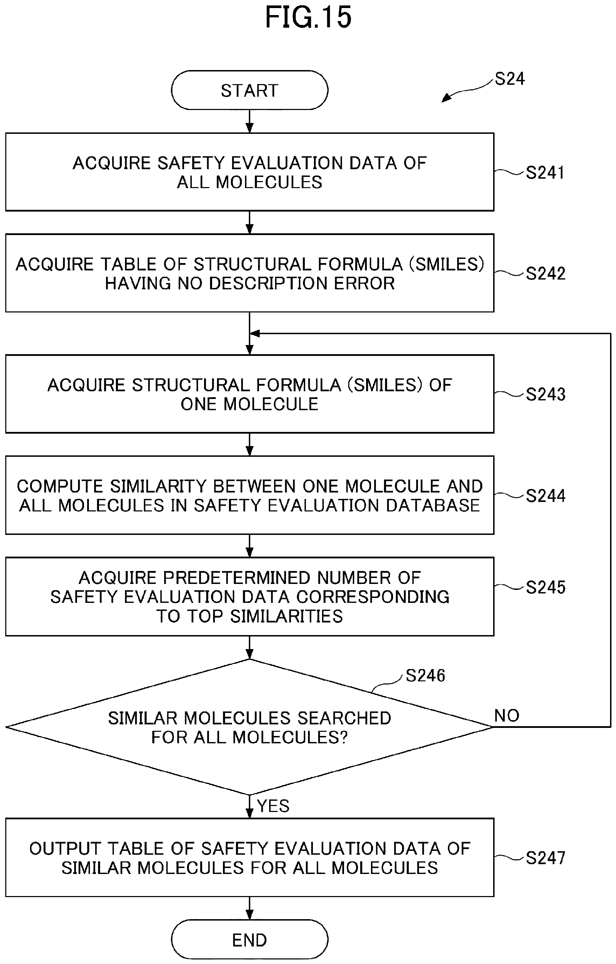

Next, the searching process (step S24) to search and acquire the safety evaluation data of the similar molecule in FIG. 12 will be described. FIG. 15 is a flow chart for explaining the searching process (step S24) to search and acquire the safety evaluation data of the similar molecule in FIG. 12. As illustrated in FIG. 15, the similar molecule data searching unit 30 of the safety prediction device 1A acquires the safety evaluation data of all of the molecules from the safety evaluation database (acquisition process to acquire the safety evaluation data of all of the molecules: step S241) .

Next, the similar molecule data searching unit 30 of the safety prediction device 1A acquires a table of structural formulas having no description error (acquisition process to acquire the table of structural formulas: step S242).

Next, the similar molecule data searching unit 30 of the safety prediction device 1A acquires the structural formula of one molecule among all of the molecules described in the table of structural formulas having no description error (acquisition process to acquire the structural formula of one molecule: step S243) .

Next, the similar molecule data searching unit 30 of the safety prediction device 1A computes the similarity between the acquired one molecule and all of the molecules in the safety evaluation database (similarity computation process: step S244).

Next, the similar molecule data searching unit 30 of the safety prediction device 1A acquires a predetermined number of safety evaluation data corresponding to top similarities, among the similarities of all of the molecules computed in the similarity computation process (step S244) (acquisition process to acquire the predetermined number of safety evaluation data: step S245).

Next, the similar molecule data searching unit 30 of the safety prediction device 1A determines whether or not the similar molecules are searched, for all of the molecules described in the table of structural formulas having no description error (determination process to determine the searched similar molecules of all of the molecules: step S246).

If the similar molecules of all of the molecules are not searched (step S246: No), the structural formula of the unchecked molecule is acquired again (step S243).

If the similar molecules of all of the molecules are searched (step S246: Yes), a table of the safety evaluation data of each of the similar molecules is output for all of the molecules (step S247).

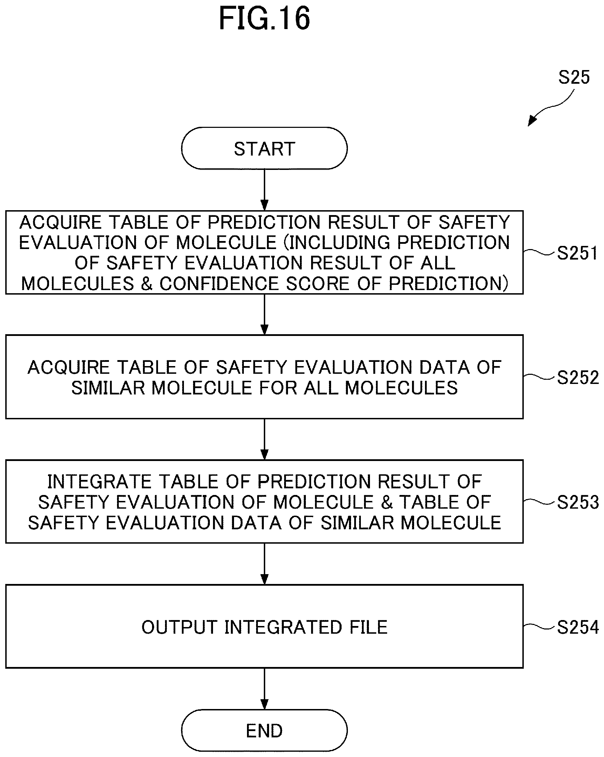

Next, the integration process (step S25) in FIG. 12 will be described. FIG. 16 is a flow chart for explaining the integration process (step S25) in FIG. 12. As illustrated in FIG. 16, the integration unit 40 of the safety prediction device 1A acquires the table of the prediction results of the safety evaluation of the molecules, including the prediction of the safety evaluation of all of the molecules and the confidence score of the prediction obtained in the computation process (step S23) to predict the safety evaluation of the molecule and compute the confidence score of the prediction, from the safety prediction unit 20 (acquisition process to acquire the table of the prediction result of the safety evaluation of the molecules: step S251).

Next, the integration unit 40 of the safety prediction device 1A acquires the table of the safety evaluation data of the similar molecule for all the molecules obtained in the acquisition process (step S24) to search and acquire the safety evaluation data of the similar molecule from the similar molecule data searching unit 30 (acquisition process to acquire the safety evaluation data of the similar molecule: step S252) .

Next, the integration unit 40 of the safety prediction device 1A integrates the table of the prediction result of the safety evaluation of the molecules and the table of the safety evaluation result of the molecules similar for all of the molecules into a single table to create an integrated file (table integration process: step S253) .

Next, the output unit 80 of the safety prediction device 1A outputs the integrated file as illustrated in FIG. 7 (integrated file output process: step S254) .

The safety prediction device 1A according to the present embodiment includes the input unit 10, the safety prediction unit 20, the similar molecule data searching unit 30, and the output unit 80. In the safety prediction device 1A, the safety prediction unit 20 predicts the safety evaluation of molecule and computes the confidence score of the prediction, and the similar molecule data searching unit 30 acquires the safety evaluation data of the similar molecule. The safety prediction device 1A can appropriately provide the user with the prediction result of the safety evaluation of the compound, by quantifying and outputting the confidence score of the prediction of the safety evaluation of the molecule. In the case where the confidence store of the prediction is high, the user can quickly and easily evaluate the safety of the compound with a high accuracy, by adopting the prediction result as it is. In the case where the confidence score of the prediction is low, the user can quickly and easily evaluate the safety of the compound with a high accuracy, quickly, by considering which of the prediction result and the safety evaluation data is to be adopted. Accordingly, the safety prediction device 1A can perform the safety evaluation of the compound with a high accuracy, while improving the convenience for the user.

The output unit 80 of the safety prediction device 1A can output a message related to the prediction result of the safety evaluation of the molecule and the confidence score of the prediction when the confidence score of the prediction is high, and output a message related to the prediction result of the safety evaluation of the molecule, the confidence score of the prediction, and the safety evaluation data when the confidence score of the prediction is low. The user can accurately determine the evaluation contents of the safety of the compound, by checking the contents of the output message. For this reason, the safety prediction device 1A can appropriately perform the safety evaluation of the compound with a high accuracy, while further improving the convenience for the user.

In the safety prediction device 1A, the safety prediction unit 20 can include the feature value computation unit 21 and the predictor 22. Hence, in the safety prediction device 1A, the feature value computation unit 21 can compute the feature value based on the structural formula of the molecule, and the predictor 22 can predict the safety of the molecule based on the computed feature value. For this reason, the safety prediction device 1A can perform the safety evaluation of the compound with an even higher accuracy.

The feature value computation unit 21 of the safety prediction device 1A can compute the feature value of the molecule by inputting the structural formula of the molecule to the characteristic prediction model 70. The safety prediction unit 20 can predict the safety evaluation of the molecule and the confidence score of the prediction with a high accuracy in a simple manner, from the structural formula of the molecule, and can reduce the load and the time required for the computation. Accordingly, the safety prediction device 1A can conveniently predict the safety evaluation of the compound with a high accuracy, and at a low computation cost.

In the safety prediction device 1A, the similar molecule data searching unit 30 may include the similarity evaluation unit 31 and the data searching unit 32. Thus, in the safety prediction device 1A, the similarity evaluation unit 31 can evaluate the similarity between the input molecules and the plurality of molecules described in the safety evaluation database 33, and the data searching unit 32 can acquire the safety evaluation data of the similar molecule having a high similarity. Accordingly, the safety prediction device 1A can perform the safety evaluation of the compound with an even higher accuracy.

The safety prediction device 1A may include the output unit 80. Hence, the safety prediction device 1A can visually present the information related to the prediction result of the safety evaluation of the predicted compound, and the information related to the similar molecule data, with respect to the user, thereby enabling the user to easily ascertain the information related to the compound.

As described above, because the safety prediction device 1A can conveniently predict the safety evaluation of the compound with a high accuracy, and at a low computation cost, the safety of the compound used in materials, pharmaceuticals, or the like utilized in chemical industry, pharmaceutical industry, or the like, for example, can be predicted with a high accuracy, and thus the safety prediction device 1A is suitable for safely performing the research and development, product manufacturing, or the like.

In addition, the safety prediction device 1A can be effectively used for an evaluation test on the biodegradability, bioaccumulation, mutagenicity, fish acute toxicity, daphnia magna acute immobilization, algal growth inhibition, mammal repeat dose toxicity, or the like. Examples of the evaluation test of the mutagenicity include a reverse mutation test (Ames test), a chromosomal aberration test, or the like. Examples of the evaluation test of the fish acute toxicity include a measurement of median lethal concentration (LC50) by "Fish Acute Toxicity Test - JIS K 0102.71 -", or the like. Examples of the evaluation test of the daphnia magna acute immobilization include a measurement of a half maximal inhibitory concentration ($EC_{50}$), or the like. Examples of the evaluation test of the algae growth inhibition include a measurement of the 50% inhibitory concentration ($EC_{50}$), or the like. Examples of the evaluation test of the mammal repeat dose toxicity include a measurement of lowest observed adverse effect level (NOAEL), or the like.

[Second Embodiment]

<Safety Prediction Device>

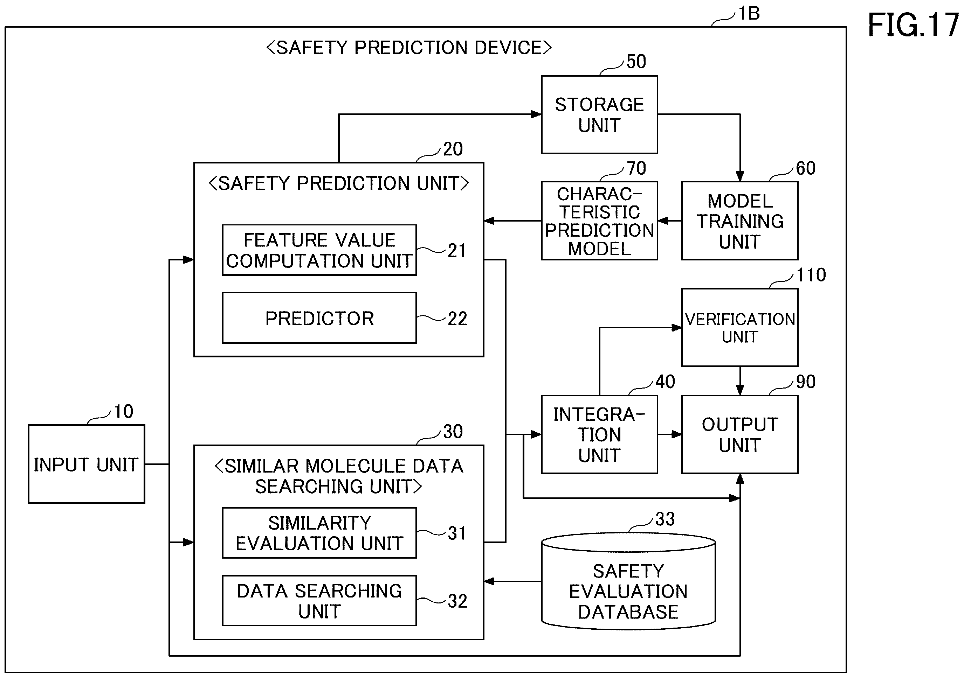

The safety prediction device according to a second embodiment of the present invention will be described. FIG. 17 is a block diagram illustrating a schematic configuration of the safety prediction device according to the present embodiment. As illustrated in FIG. 17, a safety prediction device 1B includes a verification unit 110, in addition to the configuration of the safety prediction device 1A according to the first embodiment described above. Constituent elements other than the verification unit 110 are the same as those of the safety prediction device 1A according to the first embodiment described above, and thus, a detailed description thereof will be omitted.

The verification unit 110 verifies a validity of the prediction result of the safety evaluation of the molecule, by determining a coincidence level between the prediction result of the safety evaluation of the molecule and the safety evaluation data.

In the case where the confidence score of the prediction is low, the verification unit 110 determines the coincidence level between the prediction result of the safety evaluation of the molecule and the safety evaluation data of the similar molecule. In a case where the prediction result of the safety evaluation of the molecule coincides with the safety evaluation data of the similar molecule, the verification unit 110 verifies that the prediction result is valid (OK low confidence score) although the confidence score of the prediction is low. In a case where the prediction result of the safety evaluation of the molecule does not coincide with the safety evaluation data of the similar molecule, the verification unit 110 determines that the confidence score of the prediction is low and verifies that the prediction result is not valid (NG low confidence score). The verification unit 110 refers to the safety evaluation data of the similar molecule only when the confidence score of the prediction is low, thereby reducing the frequency of use of the safety evaluation data of the similar molecule, and improving the convenience for the user.

For example, in the case where the ID in FIG. 4 is A5, it is regarded that the safety evaluation is OK, indicating easily degradable, as illustrated in FIG. 4. The confidence score of the prediction of A5 is 42%, which is less than 50%. In this case, the validity of the prediction result of the safety evaluation of the molecule may be determined by a majority decision on the safety evaluation data of a plurality of (for example, 20) similar molecules among the safety evaluation data.

In a case where the safety evaluation data of a predetermined number (for example, 11) or more similar molecules among the safety evaluation data of the plurality of (for example, 20) similar molecules coincide and the coincidence level is high, the verification unit 110 may determine that the safety evaluation of the molecule to be predicted is OK and the molecule is not easily degradable, and regard the molecule as having the OK low confidence score. In this case, the safety evaluation of the molecule to be predicted is OK, indicating easily degradable, and even when the reference is made to the safety evaluation data of the similar molecule, the safety evaluation of the molecule to be predicted is OK, indicating easily degradable, and the prediction result of the safety evaluation of the molecule obtained from the safety evaluation data and the safety evaluation data of the similar molecule coincide. For this reason, the verification unit 110 can verify that the prediction result of the safety evaluation of the molecule is valid.

On the other hand, in a case where only the safety evaluation data of less than the predetermined number of (for example, 11) similar molecules among the safety evaluation data of a plurality of (for example, 20) similar molecules coincide and the coincidence level is low, the verification unit 110 may determine that the molecule to be predicted is not easily degradable, and regard the molecule as having the NG low confidence score. In this case, the safety evaluation of the molecule to be predicted is OK, indicating easily degradable, but when a reference is made to the safety evaluation data of the similar molecule, the safety evaluation of the molecule to be predicted is NG, indicating not easily degradable, and the prediction result of the safety evaluation of the molecule and the safety evaluation data of the similar molecule do not coincide. For this reason, the verification unit 110 can verify that the prediction result of the safety evaluation of the molecule is not valid.

When determining the coincidence level between the prediction result of the safety evaluation of the molecule and the safety evaluation data of the similar molecule, the verification unit 110 may determine the coincidence level from a sum of the similarities of the similar molecules or a sum of values obtained by multiplying a weight to the similarities of the similar molecules, in place of determining the coincidence level from the majority decision on the safety evaluation data of the number of similar molecules. The weight may be the same value or different values for each of the similar molecules.

In the present embodiment, the output unit 80 may output a message indicating that the prediction result of the safety evaluation of the molecule coincides with the safety evaluation data of the similar molecule in the case where the confidence score of the prediction is low and the coincidence level is high, and output a message indicating that the prediction result of the safety evaluation of the molecule does not coincide with the safety evaluation data of the similar molecule in the case where the confidence score and the coincidence level of the prediction are low.

For example, the contents of the message may be "The confidence score of the prediction is less than 50%, but the coincidence level between the prediction result of the safety evaluation of the molecule and the safety evaluation data of the similar molecule is high." or the like in the case where the confidence score of the prediction is low and the coincidence level is high, and may be "The confidence score of the prediction is less than 50%, and the coincidence level between the prediction result of the safety evaluation of the molecule and the safety evaluation data of the similar molecule is also low." or the like in the case where the confidence score of the prediction and the coincidence level are low.

<Safety Prediction Method>

Next, a safety prediction method applied to the safety prediction device according to the present embodiment will be described. The safety prediction method applied to the safety prediction device according to the present embodiment is a method for predicting the safety of the compound using the safety prediction device 1B having a configuration illustrated in FIG. 17.

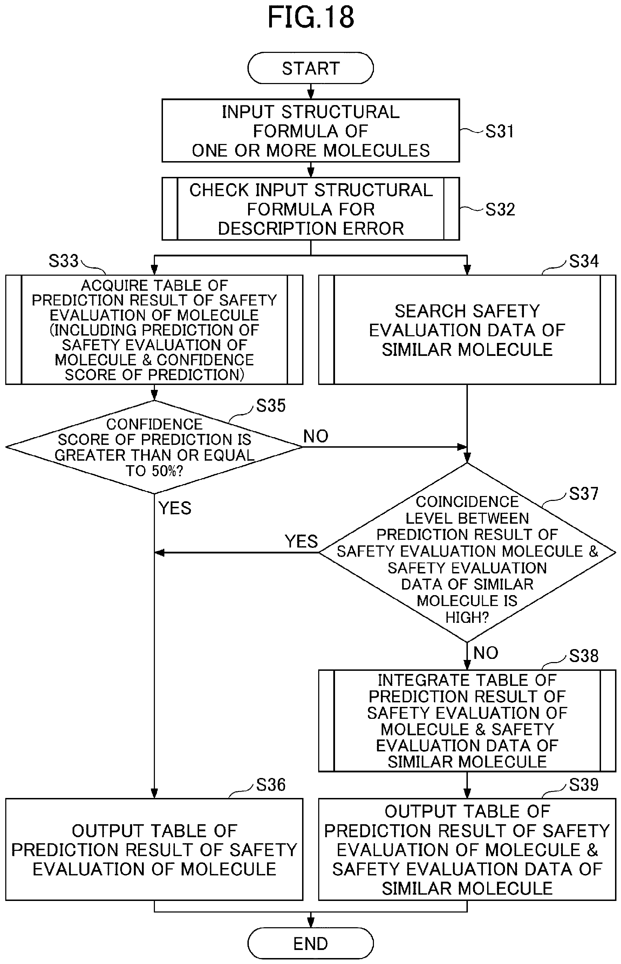

The safety prediction method applied to the safety prediction device 1B according to the present embodiment will be described. FIG. 18 is a flow chart for explaining the safety prediction method according to the present embodiment. As illustrated in FIG. 18, the input unit 10 of the safety prediction device 1B inputs the structural formula of one or more molecules to be evaluated for safety (input process: step S31).

Next, the safety prediction unit 20 of the safety prediction device 1B checks the input structural formula for a description error (checking process: step S32) .

The checking process (step S32) is the same as the checking process (step S22) of the safety prediction method according to the first embodiment illustrated in FIG. 12, and a detailed description thereof will be omitted. The checking process (step S32) may be omitted.

Next, the safety prediction unit 20 of the safety prediction device 1B predicts the safety evaluation of the molecule and computes the confidence score of the prediction, and acquires a table of the prediction result of the safety evaluation of the molecule, including the prediction of the safety evaluation of the molecule and the confidence score of the prediction (computation process to predict the safety evaluation of the molecule and compute the confidence score of the prediction: step S33).

The computation process (step S33) to predict the safety evaluation of the molecule and compute the confidence score of the prediction is the same as the computation process (step S23) to predict the safety evaluation of the molecule and compute the confidence score of the prediction of the safety prediction method according to the first embodiment illustrated in FIG. 12, and thus, a detailed description thereof will be omitted.

Next, the similar molecule data searching unit 30 of the safety prediction device 1B searches and acquires the safety evaluation data of the similar molecule similar to the molecule to be evaluated for safety (searching process to search and acquire the safety evaluation data of the similar molecule: step S34) .

The searching process (step S34) to search and acquire the similar molecules is the same as the searching process (step S24) to search and acquire the similar molecules in the safety prediction method according to the first embodiment illustrated in FIG. 12, and thus, a detailed description thereof will be omitted.

Next, the verification unit 110 of the safety prediction device 1B determines whether or not the confidence score of the prediction is greater than or equal to 50% (determination process to determine the confidence score of the prediction: step S35) after the computation process (step S33) to predict the safety evaluation of the molecule and compute the confidence score of the prediction.

In the determination process (step S35) to determine the confidence score of the prediction, if the confidence score of the prediction is greater than or equal to 50% (step S35: Yes), the output unit 80 of the safety prediction device 1B outputs the prediction result of the safety evaluation of the molecule (output process to output the prediction result: step S36).

If the confidence score of the prediction is less than 50% (step S35: No), the verification unit 110 of the safety prediction device 1B determines whether or not the coincidence level between the prediction result of the safety evaluation of the molecule and the safety evaluation data of the similar molecule is high (coincidence level determination process: step S37) after the searching process (step S34) to search and acquire the safety evaluation data of the similar molecule.

If the coincidence level between the prediction result of the safety evaluation of the molecule and the safety evaluation data of the similar molecule is high (step S37: Yes), the verification unit 110 of the safety prediction device 1B verifies that the prediction result of the safety evaluation of the molecule is valid (OK low confidence score) although the confidence score of the prediction is low, and the output unit 80 outputs the table of the prediction result of the safety evaluation of the molecule (output process to output the table of the prediction result of the safety evaluation of the molecule: step S36).

If the coincidence level between the prediction result of the safety evaluation of the molecule and the safety evaluation data of the similar molecule is low (step S37: No), the verification unit 110 of the safety prediction device 1A determines that the confidence score of the prediction is low and verifies that the prediction result of the safety evaluation of the molecule is not valid (NG low confidence score). The integration unit 40 of the safety prediction device 1A integrates the table of the prediction result of the safety evaluation of the molecule obtained in the computation process (step S33) to predict the safety evaluation of the molecule and compute the confidence score of the prediction, and the safety evaluation data of the similar molecule obtained in the searching process (step S34) to search and acquire the safety evaluation data of the similar molecule, to obtain integrated data (integration process: step S38).

The integration process (step S38) is the same as the integration process (step S25) of the safety prediction method according to the first embodiment illustrated in FIG. 12, and thus, a detailed description thereof will be omitted.

Next, the output unit 80 of the safety prediction device 1B outputs the integrated data (refer to FIG. 7) integrated by the integration unit 40 (output process: step S39).

In the safety prediction method according to the present embodiment, the computation process (step S33) to predict the safety evaluation of the molecule and compute the confidence score of the prediction may be performed simultaneously as the searching process (step S34) to search and acquire the safety evaluation data of the similar molecule, or after the searching process (step S34) to search and acquire the safety evaluation data of the similar molecule.

The safety prediction device 1B according to the present embodiment includes the verification unit 110, in addition to the configuration of the safety prediction device 1A according to the first embodiment described above, and the verification unit 110 verifies a validity of the prediction result of the safety evaluation of the molecule, determining a coincidence level between the prediction result of the safety evaluation of the molecule and the safety evaluation data. Thus, even when the confidence score of the prediction is low, the safety prediction device 1B can determine the coincidence level between the prediction result of the safety evaluation of the molecule and the safety evaluation data by making a reference to the safety evaluation data of the similar molecule, so that the safety evaluation of the compound can be performed with a high accuracy even for the compound for which the safety evaluation is difficult to predict. Accordingly, the safety prediction device 1A can perform the safety evaluation of the compound with an even higher accuracy, while further improving the convenience for the user.

The output unit 80 of the safety prediction device 1B can output a message related to the prediction result of the safety evaluation of the molecule and the confidence score of the prediction in the case where the confidence score of the prediction is high, and output a message related to the prediction result of the safety evaluation of the molecule, the confidence score of the prediction, and the safety evaluation data in the case where the confidence score of the prediction is low. Similar to the safety prediction device 1A, the user can accurately determine the evaluation of the safety of the compound, by checking the contents of the output message. Hence, the safety prediction device 1B can also appropriately perform the safety evaluation of the compound with a high accuracy, while further improving the convenience for the user.

In the safety prediction device 1B, the output unit 80 can output a message indicating that the prediction result of the safety evaluation of the molecule coincides with the safety evaluation data in a case where the confidence score of the prediction is low and the coincidence level is high, and can output a message indicating that the prediction result of the safety evaluation of the molecule does not coincide with the safety evaluation data in a case where the confidence score and the coincidence level are low. Thus, the safety prediction device 1B can provide the user with the message content indicating the coincidence level between the prediction result of the safety evaluation of the predicted compound and the safety evaluation data. The user can more accurately judge the safety evaluation of the compound by checking the contents of the output message. As a result, the safety prediction device 1B can more appropriately perform the safety evaluation of the compound, in particular, the compound for which the safety evaluation is difficult to predict, with a high accuracy, while further improving the convenience for the user.

Because the safety prediction device 1B can conveniently predict the safety evaluation of the compound with a high accuracy and at a low computation cost, similar to the safety prediction device 1A, the safety of the compound used in materials, pharmaceuticals, or the like utilized in chemical industry, pharmaceutical industry, or the like, for example, can be predicted with a high accuracy, and thus the safety prediction device 1B is suitable for safely performing the research and development, product manufacturing, or the like.

Further, similar to the safety prediction device 1A, the safety prediction device 1B can be effectively used for an evaluation test on the biodegradability, bioaccumulation, mutagenicity, fish acute toxicity, daphnia magna acute immobilization, algal growth inhibition, mammal repeat dose toxicity, or the like.

[Hardware Configuration of Safety Prediction Devices 1A and 1B]

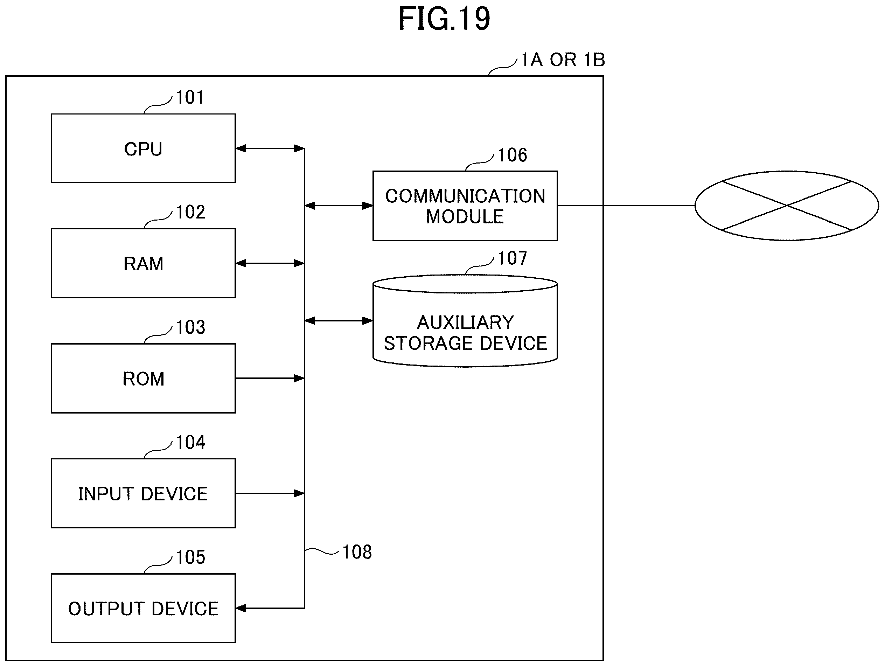

Next, an example of a hardware configuration of the safety prediction devices 1A and 1B will be described. FIG. 19 is a block diagram illustrating a hardware configuration of the safety prediction devices 1A and 1B. As illustrated in FIG. 19, the safety prediction devices 1A and 1B are configured by an information processor (computer), and can be physically configured as a computer system including a Central Processing Unit (CPU) 101 as an arithmetic unit, a Random Access Memory (RAM) 102 and a Read Only Memory (ROM) 103 as a main storage device, an input device 104, an output device 105, a communication module 106, an auxiliary storage device 107 such as a hard disk, or the like. These constituting elements of the computer system are connected to one another via a bus 108. The output device 105 and the auxiliary storage device 107 may be provided externally with respect to the computer system.

The CPU 101 controls an overall operation of the safety prediction devices 1A and 1B, and performs various information processing. The CPU 101 executes a safety prediction program stored in the ROM 103 or the auxiliary storage device 107, to control display operations of a measurement recording screen and an analyzing screen.

The RAM 102 is used as a work area of the CPU 101, and may include a non-volatile RAM that and stores main control parameters and information.

The ROM 103 stores a basic input output program or the like. The safety prediction program may be stored in the ROM 103.

The input device 104 is a keyboard, a mouse, an operation button, a touchscreen panel, or the like.

The output device 105 is a monitor display or the like. The output device 105 displays the prediction result or the like, and the screen is updated according to an input-output operation via the input device 104 or the communication module 106.

The communication module 106 is a data transmission reception device, such as a network card or the like, and functions as a communication interface that acquires information from an external data recording server or the like, and outputs analyzed information to another electronic device.

The auxiliary storage device 107 is a storage device, such as a Solid State Drive (SSD), a Hard Disk Drive (HDD), or the like, and stores various data, files, or the like required for the operation of the safety prediction devices 1A and 1B, for example.

The functions of the safety prediction devices 1A and 1B illustrated in FIG. 1 and FIG. 17 are implemented by causing the main storage device, such as the RAM 102 or the like, or the auxiliary storage device 107 of CPU 101 to read predetermined computer software (including the safety prediction program), and causing CPU 101 to execute the safety prediction program or the like stored in RAM 102, ROM 103, or the auxiliary storage device 107. The functions of the safety prediction devices 1A and 1B are implemented by operating the input device 104, the output device 105, and the communication module 106, and reading data from and writing data to the RAM 102, the ROM 103, the auxiliary storage device 107, or the like. That is, the safety prediction devices 1A and 1B can perform the functions of each of the processing units in FIG. 1 and FIG. 17, by executing the safety prediction program according to the present embodiment on the computer.

The safety prediction program is stored in the storage device included in the computer, for example. A part or all of the safety prediction program may be transmitted via a transmission medium, such as a communication line or the like, and received and recorded (including being installed) by the communication module 106 or the like included in the computer. Further, the safety prediction program may be configured to be recorded (including being installed) in the computer from a state where a part or all of the safety prediction program is stored in a portable storage medium, such as a CD-ROM, a DVD-ROM, a flash memory, or the like.

The program executed by the information processor has a module configuration including each of the processing units of the safety prediction devices 1A and 1B described above, and the processor 101 reads and executes the program as appropriate, thereby generating the processing units described above on the memory, such as the RAM 102 or the like.

The safety prediction devices 1A and 1B may be configured as a system in which a plurality of information processors are communicably connected to one another, and each of the processing units described above may be distributed among the plurality of information processors. Alternatively, the safety prediction devices 1A and 1B may be a virtual machine operating on a cloud system.

Although the present invention is described with reference to the embodiments, the present invention is not limited to the embodiments described above. The embodiments can be implemented in various other forms, and various combinations, omissions, substitutions, modifications, or the like can be made without departing from the scope of the present invention. The embodiments and modifications thereof are included in the scope and spirit of the present invention, and are also included in the scope of the present invention described in the claims and equivalents thereof.

This application is based upon and claims priority to Japanese Patent Application No. 2021-144755, filed on September 6, 2021, before the Japan Patent Office, and the entire contents of which are incorporated herein by reference.

DESCRIPTION OF REFERENCE NUMERALS

1A, 1B: compound safety prediction device; 10: input unit; 20: safety prediction unit; 21: feature value computation unit; 22: predictor; 30: similar molecule data searching unit; 31: similarity evaluation unit; 32: data searching unit; 33: safety evaluation database; 40: integration unit; 50: storage unit; 60: model training unit; 70: characteristic prediction model; 80: output unit; 110: verification unit.

**Claims**

1. A compound safety prediction device comprising:

an input unit configured to input a structural formula of one or more molecules;
a safety prediction unit configured to predict a safety evaluation of the molecule and compute a confidence score of prediction;
a similar molecule data searching unit configured to acquire safety evaluation data of a similar molecule similar to the molecule; and
an output unit configured to output a prediction result of the safety evaluation of the molecule, the confidence score of the prediction, and the safety evaluation data of the similar molecule.

2. The compound safety prediction device as claimed in claim 1, wherein the output unit outputs

a message related to the prediction result of the safety evaluation of the molecule and the confidence score of the prediction, in a case where the confidence score of the prediction is high, and
a message related to the prediction result of the safety evaluation of the molecule, the confidence score of the prediction, and the safety evaluation data of the similar molecule, in a case where the confidence score of the prediction is low.

3. The compound safety prediction device as claimed in claim 1, further comprising:
a verification unit configured to verify a validity of the prediction result of the safety evaluation of the molecule from the safety evaluation data of the similar molecule, by determining a coincidence level between the prediction result of the safety evaluation of the molecule and the safety evaluation data of the similar molecule.

4. The compound safety prediction device as claimed in claim 3, wherein the output unit outputs

a message related to the prediction result of the safety evaluation of the molecule and the confidence score of the prediction in a case where the confidence score of the prediction is high, and
a message related to the prediction result of the safety evaluation of the molecule, the confidence score of the prediction, and the safety evaluation data of the similar molecule, in a case where the confidence score of the prediction is low.

5. The compound safety prediction device as claimed in claim 4, wherein the output unit, when the confidence score of the prediction is low, outputs

a message indicating that the prediction result of the safety evaluation of the molecule coincides with the safety evaluation data of the similar molecule in a case where the coincidence level is high, and
a message indicating that the prediction result of the safety evaluation of the molecule does not coincide with the safety evaluation data of the similar molecule in a case where the coincidence level is low.

6. The compound safety prediction device as claimed in any one of claims 1 to 5, wherein the safety prediction unit includes:

a feature value computation unit configured to compute a feature value of the molecule based on the structural formula of the molecule; and
a predictor configured to predict the safety evaluation of the molecule and compute the confidence score of the prediction based on the feature value.

7. The compound safety prediction device as claimed in claim 6, wherein the feature value computation unit computes the feature value of the molecule using one or more of a fingerprint based on the structural formula of the molecule, a physical property value computed by quantum chemical computation based on the structural formula of the molecule, a physical property value obtained by a quantitative structure activity relationship between the structural formula and the physical property value of the molecule, and a prediction value obtained by a trained model that has learned the relationship between the structural formula and the physical property value of the molecule.

8. The compound safety prediction device as claimed in any one of claims 1 to 7, wherein the similar molecule data searching unit includes:

a similarity evaluation unit configured to compute a similarity between the structural formula of the molecule input by the input unit and a structural formula of a plurality of evaluated molecules in a safety evaluation database that stores safety evaluation results of the evaluated molecules evaluated in past; and

a data searching unit configured to acquire the safety evaluation result of an evaluated molecule having a high similarity as the safety evaluation data of the similar molecule.

9. A compound safety prediction program which causes a computer to execute the steps of:

inputting a structural formula of one or more molecules;

predicting a safety evaluation of the molecule and computing a confidence score of prediction;

searching to acquire safety evaluation data of a similar molecule similar to the molecule; and

outputting a prediction result of the safety evaluation of the molecule, the confidence score of the prediction, and the safety evaluation data of the similar molecule.

10. A compound safety prediction method comprising the steps of:

inputting a structural formula of one or more molecules;

predicting a safety evaluation of the molecule and computing a confidence score of prediction;

searching to acquire safety evaluation data of a similar molecule similar to the molecule; and

outputting a prediction result of the safety evaluation of the molecule, the confidence score of the prediction, and the safety evaluation data of the similar molecule.

FIG.1

# FIG.2

| ID | STRUCTURAL FORMULA (SMILES) |
|----|------------------------------|
| A1 | c1ccccc1 |
| A2 | CCCCC |
| A3 | c1ccccc1Cl |
| A4 | C1CCC |
| A5 | CCCCCCC(C)CC |

⋮

# FIG.3

| HIGH CONFIDENCE SCORE | LOW CONFIDENCE SCORE | | HIGH CONFIDENCE SCORE |
|:---:|:---:|:---:|:---:|
| NG | NG | OK | OK |

0    0.25    0.50    0.75    1.00

CLASSIFICATION PROBABILITY

# FIG.4

| ID | STRUCTURAL FORMULA (SMILES) | SAFETY EVALUATION (BOD $\geqq$ 60%) | CONFIDENCE SCORE OF PREDICTION [%] |
|---|---|---|---|
| A1 | c1ccccc1 | OK | 99 |
| A2 | CCCCC | OK | 75 |
| A3 | c1ccccc1Cl | NG | 69 |
| A4 | C1CCC | SMILES load error | SMILES load error |
| A5 | CCCCCCC(C)CC | OK | 42 |

⋮

EP 4 401 082 A1

# FIG.5

| TYPE | CHEMICAL FORMULA | CAS REGISTRY NUMBER | NAME | STRUCTURAL FORMULA (SMILES) | SAFETY EVALU-ATION (BOD) | DETERMI-NATION RESULT OF CHEMICAL SUBSTANCES CONTROL LAW | RESIDUAL CHANGE SUBSTANCE 1 | RESIDUAL CHANGE SUBSTANCE 2 | RESIDUAL CHANGE SUBSTANCE 3 | RESIDUAL CHANGE SUBSTANCE 4 | RESIDUAL CHANGE SUBSTANCE 5 | SIMILARITY |
|---|---|---|---|---|---|---|---|---|---|---|---|---|
| MOLECULE TO BE EVALUATED | ⬡—Cl | 108–90–7 | Benzene, chloro– | c1(Cl)ccccc1 | 0% | NOT EASILY DEGRADABLE | – | – | – | – | – | 1 |
| SIMILAR MOLECULE | ⬡— | 108–88–3 | Benzene, methyl– | c1(C)ccccc1 | 123% | EASILY DEGRADABLE | – | – | – | – | – | 0.538462 |
| | ⬡—OH | 108–95–2 | Phenol | c1(O)ccccc1 | 85% | EASILY DEGRADABLE | – | – | – | – | – | 0.538462 |

EP 4 401 082 A1

# FIG.6

| TYPE | CHEMICAL FORMULA | CAS REGISTRY NUMBER | NAME | STRUCTURAL FORMULA (SMILES) | SAFETY EVALUATION (BOD) | DETERMINATION RESULT OF CHEMICAL SUBSTANCES CONTROL LAW | RESIDUAL CHANGE SUBSTANCE 1 | RESIDUAL CHANGE SUBSTANCE 2 | RESIDUAL CHANGE SUBSTANCE 3 | RESIDUAL CHANGE SUBSTANCE 4 | RESIDUAL CHANGE SUBSTANCE 5 | SIMILARITY |
|---|---|---|---|---|---|---|---|---|---|---|---|---|
| MOLECULE TO BE EVALUATED | | – | – | CCCCCCC(C)CC | – | – | – | – | – | – | – | 1 |
| SIMILAR MOLECULE | | 123-96-6 | 2-Octanol | C(C)(O)CCCCCC | 76% | EASILY DEGRADABLE | – | – | – | – | – | 0.59375 |
| | | 103-44-6 | 3-[(ethenyloxy)methyl]heptane | C(CCCC)(CC)COC=C | 7% | NOT EASILY DEGRADABLE | CCCCC(CC)CO | CC=O | – | – | – | 0.35714 |

# FIG.7

| | | PREDICTION | A1 | A2 | · · · | |
|---|---|---|---|---|---|---|

# FIG.8

| CAS REGISTRY NUMBER | NAME | STRUCTURAL FORMULA (SMILES) | SAFETY EVALU-ATION (BOD) | DETERMI-NATION RESULT OF CHEMICAL SUB-STANCES CONTROL LAW | RESIDUAL CHANGE SUBSTANCE 1 | RE-SIDUAL CHANGE SUB-STANCE 2 | RE-SIDUAL CHANGE SUB-STANCE 3 | RE-SIDUAL CHANGE SUB-STANCE 4 | RE-SIDUAL CHANGE SUB-STANCE 5 |
|---|---|---|---|---|---|---|---|---|---|
| 42240-73-3 | 2,2',3,3'-tetrachloro-4, 4'-diaminodiphenylmethane | c1(N)c(Cl)c(Cl)c(Cc2c(Cl)c(Cl)c(N)cc2)cc1 | 0% | NOT EASILY DEGRAD-ABLE | CHANGE SUBSTANCE OF UNKNOWN STRUCTURE | | | | |
| 6165-51-1 | Benzene, 1, 4-dimethyl-2-(1-phenylethyl)- | c1(C)c(C(C)c2ccccc2)cc(C)cc1 | 0% | NOT EASILY DEGRAD-ABLE | − | | | | |
| 107-05-1 | 1-Propene, 3-chloro- | C(=C)CCl | 62% | EASILY DEGRAD-ABLE | − | | | | |
| 127-18-4 | Ethene, 1,1,2,2-tetrachloro- | C(Cl)(Cl)=C(Cl)Cl | 11% | NOT EASILY DEGRAD-ABLE | − | | | | |
| 760-23-6 | 1-Butene, 3,4-dichloro- | C(=C)C(Cl)CCl | 11% | NOT EASILY DEGRAD-ABLE | OC¥C=C/CO | | | | |
| 78-87-5 | Propane, 1,2-dichloro- | C(C)(Cl)CCl | 0% | NOT EASILY DEGRAD-ABLE | − | | | | |

:

EP 4 401 082 A1

# FIG.9

| ID | SAFETY EVALUATION (BOD ≧ 60%) | FEATURE VALUE 1 COMPUTED BY ECFP | FEATURE VALUE 2 COMPUTED BY ECFP | |
|----|----|----|----|----|
| 1 | NG | 0.03 | 0.07 | |
| 2 | NG | 0 | 0 | ... |
| 3 | OK | 0 | 0 | |
| 4 | OK | 0 | 0 | |

⋮

EP 4 401 082 A1

# FIG.10

TRAINING DATA
(TABLE OF
STRUCTURAL
FORMULA OF
MOLECULE OF
COMPOUND,
TABLE OF
CHARACTERISTICS
OF COMPOUND)

〈MODEL TRAINING UNIT〉 — 60

FIRST ACQUISITION UNIT — 61

TABLE OF STRUCTURAL FORMULA OF MOLECULE OF COMPOUND

SECOND ACQUISITION UNIT — 62

FUNCTION UNIT — 63

FEATURE VALUE

DETERMINATION UNIT — 64

MODEL — 65

STORAGE UNIT — 66

EP 4 401 082 A1

# FIG.11

```
          ┌─────────────┐
          │    START    │
          └──────┬──────┘
                 │
                 ▼
   ┌───────────────────────────────┐
   │    ACQUIRE TRAINING DATA      │──S11
   └───────────────┬───────────────┘
                   │
                   ▼  ◄──────────────────────┐
   ┌───────────────────────────────┐         │
   │ ACQUIRE STRUCTURAL FORMULA     │──S12    │
   │ (SMILES) OF ONE MOLECULE       │         │
   └───────────────┬───────────────┘         │
                   │                          │
                   ▼                          │
   ┌───────────────────────────────┐         │
   │    COMPUTE FEATURE VALUE       │──S13    │
   └───────────────┬───────────────┘         │
                   │                          │
                   ▼       S14                │
              ◇───────────────◇   NO          │
             FEATURE VALUE OF   ├─────────────┘
          ALL MOLECULES COMPUTED?
              ◇───────────────◇
                   │ YES
                   ▼
   ┌───────────────────────────────┐
   │            TRAIN              │──S15
   └───────────────┬───────────────┘
                   │
                   ▼
   ┌───────────────────────────────┐
   │          STORE MODEL          │──S16
   └───────────────┬───────────────┘
                   │
                   ▼
          ┌─────────────┐
          │     END     │
          └─────────────┘
```

# FIG.12

START

↓

INPUT STRUCTURAL FORMULA OF ONE OR MORE MOLECULES — S21

↓

CHECK INPUT STRUCTURAL FORMULA FOR DESCRIPTION ERROR — S22

↓

S23

ACQUIRE TABLE OF PREDICTION RESULT OF SAFETY EVALUATION OF MOLECULE (INCLUDING PREDICTION OF SAFETY EVALUATION OF MOLECULE & CONFIDENCE SCORE OF PREDICTION)

S24

SEARCH SAFETY EVALUATION DATA OF SIMILAR MOLECULE

↓

INTEGRATE TABLE OF PREDICTION RESULT OF SAFETY EVALUATION OF MOLECULE & SAFETY EVALUATION DATA OF SIMILAR MOLECULE — S25

↓

OUTPUT — S26

↓

END

# FIG.13

```
                                              S22
         START

            │
            ▼
┌──────────────────────────────────┐
│  INPUT STRUCTURAL FORMULA (SMILES) OF │  S221
│  ALL MOLECULES TO BE EVALUATED    │
└──────────────────────────────────┘
            │
            ▼  ◄──────────────────────────┐
┌──────────────────────────────────┐      │
│  ACQUIRE STRUCTURAL FORMULA (SMILES) OF │  S222 │
│  ONE MOLECULE                     │      │
└──────────────────────────────────┘      │
            │                              │
            ▼                              │
┌──────────────────────────────────┐      │
│  CHECK FOR DESCRIPTION ERROR IN STRUCTURAL │  S223 │
│  FORMULA (SMILES) OF MOLECULE     │      │
└──────────────────────────────────┘      │
            │                              │
            ▼           S224               │
         ╱        ╲                   NO   │
      ╱  DESCRIPTION ERROR CHECKED  ╲──────┘
      ╲  FOR ALL MOLECULES?         ╱
         ╲        ╱
            │ YES
            ▼
┌──────────────────────────────────┐
│  OUTPUT TABLE OF STRUCTURAL FORMULA (SMILES) │  S225
│  HAVING NO DESCRIPTION ERROR TO FILE │
└──────────────────────────────────┘
            │
            ▼
┌──────────────────────────────────┐
│  OUTPUT TABLE OF STRUCTURAL FORMULA (SMILES) │  S226
│  HAVING DESCRIPTION ERROR TO FILE │
└──────────────────────────────────┘
            │
            ▼
          END
```

# FIG.14

START — S23

↓

ACQUIRE CHARACTERISTIC PREDICTION MODEL — S231

↓

ACQUIRE TABLE OF STRUCTURAL FORMULA (SMILES) HAVING NO DESCRIPTION ERROR — S232

↓

ACQUIRE STRUCTURAL FORMULA (SMILES) OF ONE MOLECULE — S233

↓

GENERATE FEATURE VALUE OF ONE MOLECULE — S234

↓

PREDICT SAFETY EVALUATION & COMPUTE CONFIDENCE SCORE OF PREDICTION — S235

↓

PREDICTION OF SAFETY EVALUATION & COMPUTATION OF CONFIDENCE SCORE OF PREDICTION PERFORMED FOR ALL MOLECULES? — S236 — NO

↓ YES

OUTPUT TABLE OF PREDICTION RESULT OF SAFETY EVALUATION OF MOLECULE — S237

↓

END

# FIG.15

```
                    START                         ╱S24

          ACQUIRE SAFETY EVALUATION DATA OF        S241
                   ALL MOLECULES

        ACQUIRE TABLE OF STRUCTURAL FORMULA (SMILES)   S242
             HAVING NO DESCRIPTION ERROR

          ACQUIRE STRUCTURAL FORMULA (SMILES) OF    S243
                    ONE MOLECULE

        COMPUTE SIMILARITY BETWEEN ONE MOLECULE AND   S244
       ALL MOLECULES IN SAFETY EVALUATION DATABASE

          ACQUIRE PREDETERMINED NUMBER OF          S245
        SAFETY EVALUATION DATA CORRESPONDING
                 TO TOP SIMILARITIES

                                                  S246
             SIMILAR MOLECULES SEARCHED            NO
                 FOR ALL MOLECULES?

                        YES

        OUTPUT TABLE OF SAFETY EVALUATION DATA OF   S247
         SIMILAR MOLECULES FOR ALL MOLECULES

                      END
```

# FIG.16

S25

START

↓

ACQUIRE TABLE OF PREDICTION RESULT OF SAFETY EVALUATION OF MOLECULE (INCLUDING PREDICTION OF SAFETY EVALUATION RESULT OF ALL MOLECULES & CONFIDENCE SCORE OF PREDICTION) ~S251

↓

ACQUIRE TABLE OF SAFETY EVALUATION DATA OF SIMILAR MOLECULE FOR ALL MOLECULES ~S252

↓

INTEGRATE TABLE OF PREDICTION RESULT OF SAFETY EVALUATION OF MOLECULE & TABLE OF SAFETY EVALUATION DATA OF SIMILAR MOLECULE ~S253

↓

OUTPUT INTEGRATED FILE ~S254

↓

END

FIG.17

# FIG.18

START

INPUT STRUCTURAL
FORMULA OF
ONE OR MORE MOLECULES — S31

CHECK INPUT STRUCTURAL
FORMULA FOR
DESCRIPTION ERROR — S32

S33
ACQUIRE TABLE OF
PREDICTION RESULT OF SAFETY
EVALUATION OF MOLECULE
(INCLUDING PREDICTION OF
SAFETY EVALUATION OF
MOLECULE & CONFIDENCE
SCORE OF PREDICTION)

S34
SEARCH SAFETY
EVALUATION DATA OF
SIMILAR MOLECULE

S35
CONFIDENCE
SCORE OF PREDICTION IS
GREATER THAN OR EQUAL
TO 50%?   NO

YES

S37
COINCIDENCE
LEVEL BETWEEN
PREDICTION RESULT OF
SAFETY EVALUATION MOLECULE &
SAFETY EVALUATION
DATA OF SIMILAR
MOLECULE IS
HIGH?

YES

NO

S38
INTEGRATE TABLE OF
PREDICTION RESULT OF
SAFETY EVALUATION OF
MOLECULE & SAFETY
EVALUATION DATA OF
SIMILAR MOLECULE

S36
OUTPUT TABLE OF
PREDICTION RESULT OF SAFETY
EVALUATION OF MOLECULE

S39
OUTPUT TABLE OF
PREDICTION RESULT OF SAFETY
EVALUATION OF MOLECULE &
SAFETY EVALUATION DATA OF
SIMILAR MOLECULE

END

# FIG.19

## INTERNATIONAL SEARCH REPORT

| International application No. |
|---|
| **PCT/JP2022/032725** |

**A. CLASSIFICATION OF SUBJECT MATTER**

*G16C 20/30*(2019.01)i; *G16C 20/40*(2019.01)i; *G06F 16/907*(2019.01)i
FI: G16C20/30; G16C20/40; G06F16/907

According to International Patent Classification (IPC) or to both national classification and IPC

**B. FIELDS SEARCHED**

Minimum documentation searched (classification system followed by classification symbols)

G16C20/00-20/90; G06F16/00-16/958

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2007-153767 A (THE UNIVERSITY OF TOKUSHIMA) 21 June 2007 (2007-06-21) | 1-10 |
| A | KR 10-2020-0072585 A (LEE, Yul Hee) 23 June 2020 (2020-06-23) | 1-10 |
| A | WO 2009/025045 A1 (FUJITSU LTD.) 26 February 2009 (2009-02-26) | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 November 2022** | **15 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/032725**

| Patent document cited in search report | | | Publication date (day/month/year) | Patent family member(s) | | | Publication date (day/month/year) |
|---|---|---|---|---|---|---|---|
| JP | 2007-153767 | A | 21 June 2007 | (Family: none) | | | |
| KR | 10-2020-0072585 | A | 23 June 2020 | (Family: none) | | | |
| WO | 2009/025045 | A1 | 26 February 2009 | US<br>EP | 2010/0145896<br>2180435 | A1<br>A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5512077 B
- JP 2007153767 A
- JP 2021144755 A